# EUROPEAN PATENT APPLICATION

(11) **EP 4 506 421 A1**
(43) Date of publication of application: **12.02.2025**
(21) Application number: 23784689.4
(22) Date of filing: 29.03.2023
(51) Int. Cl.: C08L 101/00, A61K 8/02, A61K 8/73, A61K 8/89, A61Q 1/00, A61Q 19/00, C08J 3/12, C08K 3/013, C08L 1/00, C08L 21/00, C08L 83/04

(54) **COMPOSITE PARTICLES, METHOD FOR PRODUCING COMPOSITE PARTICLES, AND COSMETIC**

(30) Priority: 08.04.2022 JP 2022064734
(71) Applicant: SHIN-ETSU CHEMICAL CO., LTD., Tokyo 1000005 (JP)
(72) Inventor: INOKUCHI Yoshinori, Annaka-shi, Gunma 379-0224 (JP); TANAKA Mitsuru, Tokyo 100-0005 (JP)
(74) Representative: Sonnenhauser, Thomas Martin
(86) International application number: PCT/JP2023/012985
(87) International publication number: WO 2023/195402

(57) **Abstract**

The present invention relates to a composite particle including an organic resin particle having a spherical shape and a rubber particle adhered on the surface of the organic resin particle, wherein the rubber particle is fixed to the organic resin particle using silica as a binder. The present invention provides: the composite particle having the rubber particle adhered on the surface of the organic resin particle, the composite particle that can give the effect of a soft feeling to the touch, a smooth feeling in use, spreadability, and a natural finish thanks to light scattering properties; a manufacturing method for the composite particles; and a cosmetic including the composite particles.

## Description

### TECHNICAL FIELD

The present invention relates to: a composite particle, specifically a composite particle comprising an organic resin particle having a rubber particle adhered on a surface of the organic resin particle; a manufacturing method for the composite particle; and cosmetic comprising the particle.

### BACKGROUND ART

Spherical particles of organic resins (plastics) such as polyamide resins, polyacrylic resins, polystyrene resins, and silicone resins (polyorganosilsesquioxanes) are used in cosmetics such as foundations to provide a smooth feeling in use and spreadability. Furthermore, because they scatter light, they can also provide a natural finish without an unnatural shine. These particles are made of resin (plastic), and therefore, they have the disadvantage of being hard to the touch.

Microplastics, which are water-insoluble, solid, and microscopic organic substances with a diameter of 5 mm or less, are a worldwide high concern in itself due to concerns about their impact on the ecosystem including a risk that the chemical substances they contain or adsorb may be introduced into the food chain, **etc.,** and authorities in some countries are considering regulation on them. Because microplastics manufactured as particles as described above are very small in size, when they flow into rivers, oceans, ponds, **etc.,** it is difficult to recover them. Furthermore, the organic resin particles described above decompose very slowly in the natural environment. Therefore, it has been proposed to use cellulose particles derived from nature-based products for cosmetics (Patent Document 1). However, although cellulose particles are biodegradable, they have the disadvantage of low slipperiness and being hard to the touch.

Patent Document 2 proposes a cosmetic using a composite particle having a silicone elastomer adhered on the surface of the particle (base particle). The light scattering properties of the silicone elastomer are said to improve a natural finish and provide a soft, moist feeling to the touch. In order to fix the silicone elastomer to the particle surface, Patent Document 2 specifically exemplifies that a silicone resin (polyorganosilsesquioxane) is used as a binder. When the base particle of the composite particle is an organic resin, the slip properties may be inferior to those when the base particle is an inorganic particle, and furthermore, stretchability and spreadability may get worse, resulting in twisting. This is considered to be due to that the organic resin as the base particle and the silicone resin as the binder are not as hard as the inorganic particle.

### CITATION LIST

### PATENT LITERATURE

Patent Document 1: WO2020/004604 A1 pamphlet
Patent Document **2:** JP 2011-1332 A

### SUMMARY OF INVENTION

### TECHNICAL PROBLEM

The present invention has been made in view of the above-described problem. An object of the present invention is to provide: a composite particle comprising an organic resin particle having a rubber particle adhered on a surface of the organic resin particle, the composite particle that can give effect of a soft feeling to the touch, a smooth feeling in use, spreadability, a natural finish thanks to light scattering properties; a manufacturing method for the composite particle; and cosmetic comprising the composite particle.

### SOLUTION TO PROBLEM

To solve the above problem, the present invention provides a composite particle comprising: an organic resin particle having a spherical shape; and a rubber particle adhered on a surface of the organic resin particle,
wherein the rubber particle is fixed to the organic resin particle using silica as a binder.

Such a composite particle can give effect of a soft feeling to the touch, a smooth feeling in use, spreadability, and a natural finish thanks to light scattering properties.

Further, content of the rubber particle is preferably within a range of 0.1 to 100 parts by mass relative to 100 parts by mass of the organic resin particle.

A cosmetic with such a content of the rubber particle can give effect of a remarkably softer feeling to the touch, a smoother feeling in use, spreadability, and light scattering properties, and can have low aggregativity.

Further, the content of the silica is preferably within the range of 10 to 1,000 parts by mass relative to 100 parts by mass of the rubber particle.

With such content of the silica, it is possible to make the rubber particle fix on a surface of the organic resin particle more surely and give a cosmetic a soft feeling to the touch.

Further, the rubber particle is preferably a silicone rubber particle.

In the present invention, such a rubber particle can be preferably used.

Further, the organic resin particle is preferably a cellulose particle.

In the present invention, such an organic resin particle can be preferably used.

Further, the present invention provides a method for manufacturing the aforementioned composite particle,
the method comprising adding tetraalkoxysilane to a liquid mixture of the organic resin particle, the rubber particle, a cationic substance, an alkaline substance, and water, and subjecting the tetraalkoxysilane to a hydrolysis/condensation reaction,
wherein the cationic substance is a cationic surfactant and/or a cationic water-soluble polymer.

According to such a method, it is possible to manufacture the inventive composite particle.

Further, the cationic substance is preferably a cationic surfactant and a cationic water-soluble polymer.

According to such a method, it is possible to manufacture the inventive composite particle more surely.

Further, the blending amount of the cationic substance is preferably within the range of 0.0001 to 2.0 parts by mass relative to 100 parts by mass of the water in the liquid mixture.

According to such a method, the rubber particle adheres to the surface of the organic resin particle, and fixing by silica becomes sufficient.

Further, the blending amount of the cationic surfactant is preferably within the range of 0.001 to 1.9 parts by mass relative to 100 parts by mass of the water in the liquid mixture, and the blending amount of the cationic water-soluble polymer is preferably within the range of 0.0001 to 1.0 parts by mass relative to 100 parts by mass of the water in the liquid mixture.

According to such a method, the rubber particle adheres to the surface of the organic resin particle more surely, and fixing by silica becomes sufficient.

Further, the present invention provides a cosmetic comprising the aforementioned composite particle.

The inventive cosmetic has a soft feeling to the touch, a smooth feeling in use, spreadability, and a natural finish thanks to light scattering properties.

### ADVANTAGEOUS EFFECTS OF INVENTION

The present invention can provide a composite particle that can give effect of a soft feeling to the touch, a smooth feeling in use, spreadability, and a natural finish thanks to light scattering properties, a manufacturing method for the same, and a cosmetic containing the composite particle.

### BRIEF DESCRIPTION OF DRAWINGS

FIG. 1 is an electron micrograph of a composite particle obtained in Example 1;
FIG. 2 is an electron micrograph of a composite particle obtained in Example 2;
FIG. 3 is an electron micrograph of a composite particle obtained in Example 3;
FIG. 4 is an electron micrograph of a cellulose particle used in Examples and Comparative Examples; and
FIG. 5 shows the measurement results of oil absorption of composite particles of Examples and cellulose particles of Comparative Examples.

### DESCRIPTION OF EMBODIMENTS

As described above, it has been desired to develop a composite particle comprising an organic resin particle having a rubber particle adhered on a surface of the organic resin particle, the composite particle that can give the effect of a soft feeling to the touch, a smooth feeling in use, spreadability, and a natural finish thanks to light scattering properties, a manufacturing method for the same, and a cosmetic containing the composite particle.

As a result of their diligent study of the above problems, the inventors found a composite particle comprising: an organic resin particle having a spherical shape; and a rubber particle fixed on a surface of the organic resin particle using silica as a binder, can give a cosmetic the effect of a soft feeling to the touch, a smooth feeling in use, spreadability, and a natural finish thanks to light scattering properties, and have completed the present invention.

That is, the present invention relates to a composite particle comprising: an organic resin particle having a spherical shape; and a rubber particle adhered on a surface of the organic resin particle, wherein the rubber particle is fixed to the organic resin particle using silica as a binder.

Further, the present invention relates to a method for manufacturing the aforementioned composite particle, the method comprising adding tetraalkoxysilane to a liquid mixture of the organic resin particle, the rubber particle, a cationic substance, an alkaline substance, and water, and subjecting the tetraalkoxysilane to a hydrolysis/condensation reaction, wherein the cationic substance is a cationic surfactant and/or a cationic water-soluble polymer.

Hereinafter, the present invention will be specifically described, but the present invention is not limited thereto.

### [Composite Particle]

The inventive composite particle comprises: an organic resin particle having a spherical shape; and a rubber particle adhered on a surface of the organic resin particle, wherein the rubber particle is fixed to the organic resin particle using silica as a binder.

The volume-average particle diameter of the particle can be measured according to a coulter counter method (an electrical resistance method). The particle diameter depends on the diameter of the organic resin particle, the diameter of the rubber particle, and the number of the rubber particle adhered on the surface of the organic resin particle.

### [Organic Resin Particle]

The organic resin particle used for the present invention is a particle that is the core of the composite particle. One kind of the organic resin particles may be used alone, or two or more kinds thereof may be used in an appropriate combination. It is possible to use any powder that can be substantially used for cosmetics regardless of its particle diameter, as long as the particle has a spherical shape.

The shape of the organic resin particle is spherical. In this specification, "spherical" means not only a true sphere, but also a deformed sphere in which the average "length of the longest axis/length of the shortest axis (aspect ratio)" is usually 1.0 to 4.0, preferably in the range 1.0 to 2.0, more preferably 1.0 to 1.6, further preferably 1.0 to 1.4. The shape of the particle can be confirmed, for example, by observing the particle using an optical microscope, an electron microscope, etc. The structure of the particle may be either non-porous or porous.

The volume-average particle diameter is preferably within the range of 0.5 to 50 µm, more preferably within the range of 1 to 30 µm. When the particle diameter is 0.5 µm or more, it is possible to obtain sufficient effect to give a dry or smooth feeling in use and spreadability. When the particle diameter is 50 µm or less, it is possible to reduce a rough feeling. Note that, the volume-average particle diameter can be measured according to a coulter counter method (an electrical resistance method).

Examples of the organic resin particle include a particle of polyethylene; polypropylene; polystyrene; divinylbenzene resin; polyvinyl chloride; methacrylic resin; polytetrafluoroethylene; a methacrylic-styrene copolymer; polyamide; polycarbonate; polyesters such as polyethylene terephthalate, polybutylene succinate, polyhydroxybutyric acid, and polycaprolactone; cellulose; cellulose derivatives; calcium alginate; phenolic resin; melamine resin; benzoguanamine resin; epoxy resin; and polyurethane, etc.

When biodegradable properties are desired, examples thereof include the particle of: polybutylene succinate; polyhydroxybutyrate; polycaprolactone; cellulose; and cellulose derivatives such as cellulose acetate and cellulose acetate propionate. Furthermore, when a particle derived from a natural product is desired, examples thereof include cellulose particles.

### [Rubber Particle]

The rubber particle used for the present invention is a particle adhered to the surface of the organic resin particle being the core of the composite particle. One kind of the rubber particle can be used alone, or two or more kinds thereof may be used in combination, and it is possible to use any powder that can be substantially used for a cosmetic regardless of its particle diameter. In addition, as long as the geometric form is one that is usually used in cosmetics, it may be any shape such as spherical, polyhedral, spindle, needle, plate, etc., and may be either non-porous or porous.

The volume-average particle diameter of the rubber particle is preferably smaller than that of the organic resin particle. When smaller than those of the organic resin particle being the core of powder, it is possible to exhibit powder properties such as a soft feeling to the touch, a smooth feeling in use, spreadability, and light scattering properties sufficiently. The volume-average particle diameter is preferably within the range of 0.05 to 5 µm, more preferably within the range of 0.1 to 1 µm. Note that, the volume-average particle diameter can be measured according to a laser diffraction/scattering method.

The rubber constituting the rubber particle is preferably non-sticky, and its rubber hardness is preferably in the range of 5 to 95, more preferably 10 to 70, when measured by a type A durometer specified in JIS K6253. When the rubber hardness is 5 or more, the aggregability is low, and good slipperiness and spreadability can be given to a cosmetic so that it does not become twisted. When the rubber hardness is 95 or less, a soft feeling to the touch can be obtained. Note that, the rubber hardness is the value by measurement in which test pieces according to the composition of the particle were prepared in accordance with the shape and dimensions specified in JIS K6253.

Examples of the rubber particle include a particle of polybutadiene rubber, acrylic rubber, urethane rubber, silicone rubber, fluororubber, etc. Silicone rubber particles are preferable because they provide a soft feeling to the touch easily.

The silicone rubber preferably includes a cured product having linear organosiloxane blocks represented by the formula "-(R₂SiO₂/₂)ₐ-", wherein R is a substituted or unsubstituted monovalent hydrocarbon group having 1 to 30 carbon atoms, and "a" is a positive integer of 5 to 5,000.

R has 1 to 30 carbon atoms, preferably 1 to 22, and more preferably 1 to 18. Examples of R include alkyl groups such as a methyl group, an ethyl group, a propyl group, a butyl group, a pentyl group, a hexyl group, a heptyl group, an octyl group, a decyl group, a undecyl group, a dodecyl group, a tetradecyl group, a pentadecyl group, a hexadecyl group, a heptadecyl group, a octadecyl group, a nonadecyl group, an icosyl group, a henicosyl group, a docosyl group, a tricosyl group, a tetracosyl group, and a triacontyl group; aryl groups such as a phenyl group, a tolyl group, and a naphthyl group; aralkyl groups such as a benzyl group and a phenethyl group; alkenyl groups such as a vinyl group and an allyl group; cycloalkyl groups such as a cyclopentyl group, a cyclohexyl group, and a cycloheptyl group; and hydrocarbon groups in which some or all of the hydrogen atoms bonded to a carbon atom of these groups are substituted with atoms such as halogen atoms (a fluorine atom, a chlorine atom, a bromine atom, or an iodine atom) and/or substituents such as a acryloyloxy group, a methacryloyloxy group, an epoxy group, a glycidoxy group, and a carboxyl group. They are preferably an alkyl group having 1 to 18 carbon atoms or a phenyl group, and it is preferable that 50 mol% or more of all R groups is a methyl group.

The silicone rubber is obtained from a curable liquid silicone composition, and its curing can be exemplified by an addition reaction, a condensation reaction, a radical reaction, etc. The curable liquid silicone composition contains a component having a reactive group for the above curing reaction (one having both reactive groups, or a component having one reactive group and a component having the other reactive group), and a curing catalyst or a radical generator.

When a silicone rubber is manufactured by curing through an addition reaction, the curable liquid silicone composition can be exemplified by one that contains (A) organopolysiloxane having an alkenyl group, (B) organohydrogenpolysiloxane having a silicon-bonded hydrogen atom, and a catalyst for addition reaction.

Component (A) is an organopolysiloxane represented by the following average composition formula (1) having at least two alkenyl groups in one molecule.

R¹_{b}R²_{c}SiO_{(4-b-c)/2} (1)

In the formula, R¹ is each independently a monovalent hydrocarbon group having 1 to 30 carbon atoms that is unsubstituted or substituted and does not contain an alkenyl group. R² is each independently an alkenyl group having 2 to 6 carbon atoms. "b" and "c" are positive numbers that satisfy 0<b<3, 0<c≤3, and 0.1≤b+c≤3.

One kind of the organopolysiloxanes represented by the average composition formula (1) may be used alone, or two or more kinds thereof may be used in combination.

The number of carbon atoms of R¹ is 1 to 30, preferably 1 to 22, and more preferably 1 to 18. R¹ may be exemplified by monovalent hydrocarbon groups excluding an alkenyl group among those listed above for R, and it is preferable that 50 mol% or more of R¹ is a methyl group. R² may be exemplified by a vinyl group, an allyl group, a propenyl group, a butenyl group, a pentenyl group, or a hexenyl group, and is preferably a vinyl group. "b" and "c" are preferably positive numbers satisfying 0<b≤2.295, 0.005≤c≤2.3, and 0.5≤b+c≤2.3.

The viscosity of component (A) at 25°C is preferably 100,000 mm²/s or less, more preferably 10,000 mm²/s or less. When the viscosity is 100,000 mm²/s or less, it is particularly easy to obtain a silicone microparticle with a narrow particle diameter distribution in preparation of an aqueous dispersion of the silicone rubber particles described later. The lower limit of the viscosity is not particularly limited, but it may be 0.7 mm²/s or more, particularly 2 mm²/s or more. In the present invention, the value of the kinetic viscosity may be the value measured using a capillary viscometer at 25°C.

Although the structure of component (A) may be linear, cyclic, or branched, it is preferable to be linear or branched structures having fewer branch units. There is no particular restriction on the bonding sites of the alkenyl groups, and they may be bonded to any silicon atoms on either the side chain or the terminals of the molecule, it is particularly preferable to bond to silicon atoms at both terminals of the linear organopolysiloxane.

Examples of the linear structure are represented by the following general formula (2).

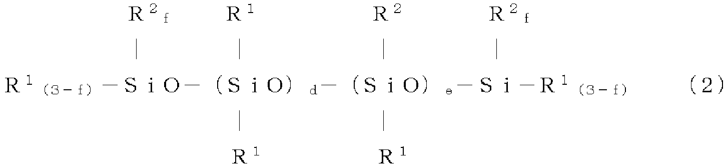

In the formula, R¹ and R² are the same as above, "d" is a positive number, "e" is 0 or a positive number, and "f" is 0, 1, 2, or 3, provided that "e" and "f" are numbers that satisfy e+2×f≥2.

Examples of the branched structure include those represented by the following general formula (3) which is branched from R¹SiO_{3/2} units.

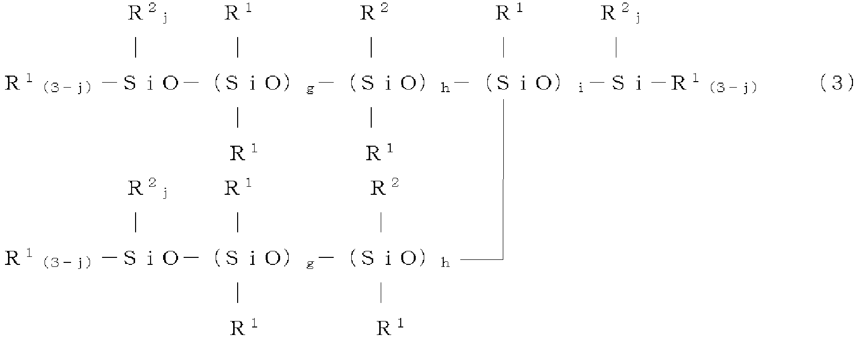

In the formula, R¹ and R² are the same as above, "g" is a positive number, "h" is 0 or a positive number, "i" is a positive number, and "j" is 0, 1, 2, or 3, provided that "h" and "j" are numbers that satisfy h+j≥1.

Examples of the structure branched from SiO_{4/2} units include those represented by the following general formula (4).

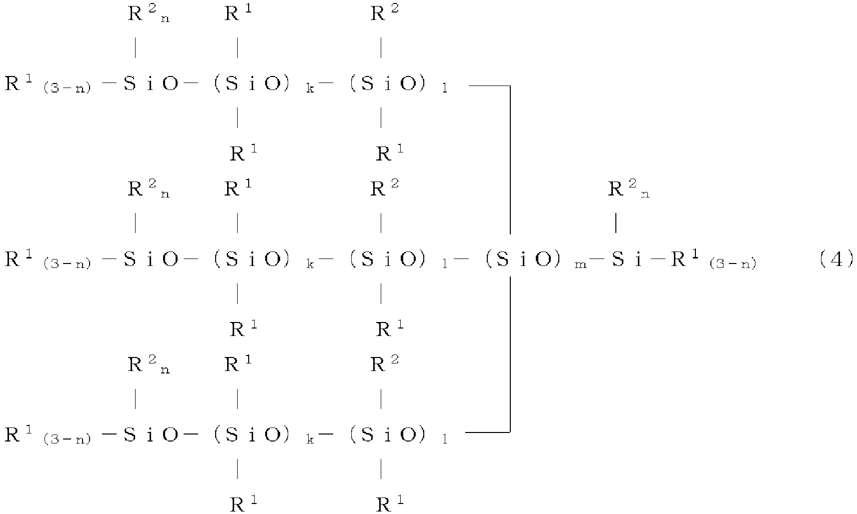

In the formula, R¹ and R² are the same as defined above, "k" is a positive number, "l" is 0 or a positive number, "m" is a positive number, and "n" is 0, 1, 2, or 3, provided that "l" and "n" are numbers that satisfy l+n≥1.

Further, examples include those having two or more alkenyl groups per molecule and represented by the following unit formula (5).

[R¹₃SiO_{1/2}]ₒ[R²(R¹)₂SiO_{1/2}]ₚ[SiO_{4/2}]_{q}[(OR³)SiO_{3/2}]ᵣ (5)

In the formula, R¹ and R² are the same as defined above, R³ is a hydrogen atom or an unsubstituted monovalent hydrocarbon group having 1 to 6 carbon atoms, "o" is 0 or a positive number, "p" is a positive number, "q" is a positive number, and "r" is 0 or a positive number.

Component (B) is an organohydrogenpolysiloxane having at least two hydrogen atoms bonded to a silicon atom (referred to as an SiH group) in one molecule and represented by the following average composition formula (6).

R⁴ₛHₜSiO_{(4-s-t)/2} (6)

In the formula, R⁴ is each independently an unsubstituted or substituted monovalent hydrocarbon group that has 1 to 30 carbon atoms and does not contain an alkenyl group, and "s" and "t" are numbers that satisfy 0<s<3, 0<t≤3, and 0.1≤s+t≤3.

One kind of the organopolysiloxanes represented by the average composition formula (6) may be used alone, or two or more kinds thereof may be used in combination.

The number of carbon atoms of R⁴ is 1 to 30, preferably 1 to 22, and more preferably 1 to 18. R⁴ may be the same monovalent hydrocarbon group as R¹, and preferably 80 mol% or more of R⁴ is a methyl group, more preferably 95% or more of R⁴ is a methyl group. "s" and "t" are preferably positive numbers satisfying 0<s≤2.295, 0.005≤t≤2.3, and 0.5≤s+t≤2.3.

The viscosity of component (B) at 25°C is preferably 100,000 mm²/s or less, more preferably 10,000 mm²/s or less. When the viscosity is 100,000 mm²/s or less, it is particularly easy to obtain a silicone microparticle with a narrow particle diameter distribution in preparation of an aqueous dispersion of silicone rubber particles described later. The lower limit of the viscosity is not particularly limited, but it may be 0.4 mm²/s or more, particularly 2 mm²/s or more.

The structure of component (B) may be linear, cyclic, or branched, but it is particularly preferable to be linear or branched. There is no particular restriction on the bonding sites of the hydrogen atoms bonded to a silicon atom, and they may be bonded to any silicon atom on either the side chain or the terminals of the molecule.

Examples of the linear structure include those represented by the following general formula (7).

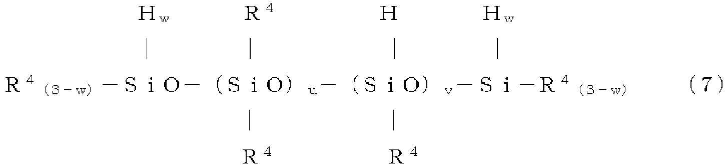

In the formula, R⁴ is the same as defined above, "u" is a positive number, "v" is 0 or a positive number, and "w" is 0, 1, 2, or 3, provided that "v" and "w" are numbers that satisfy v+2×w≥2.

Examples of the branched structure include those represented by the following general formula (8) that is branched from R⁴SiO_{3/2} units.

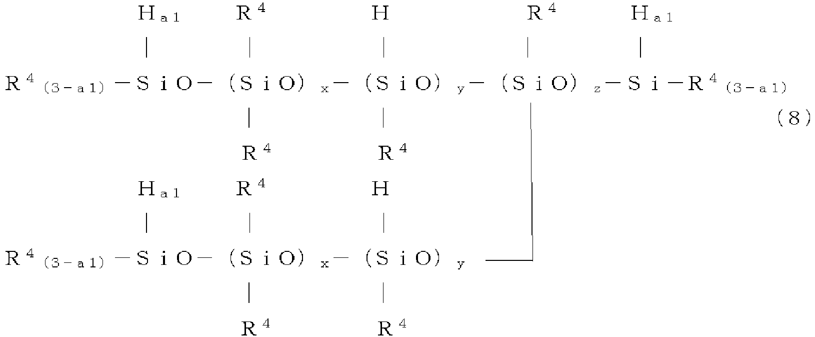

In the formula, R⁴ is the same as defined above, "x" is a positive number, "y" is 0 or a positive number, "z" is a positive number, and "a1" is 0, 1, 2, or 3, provided that "y" and "a1" are numbers that satisfy y+a1≥1.

Examples of a structure branched from SiO_{4/2} units include those represented by the following general formula (9). 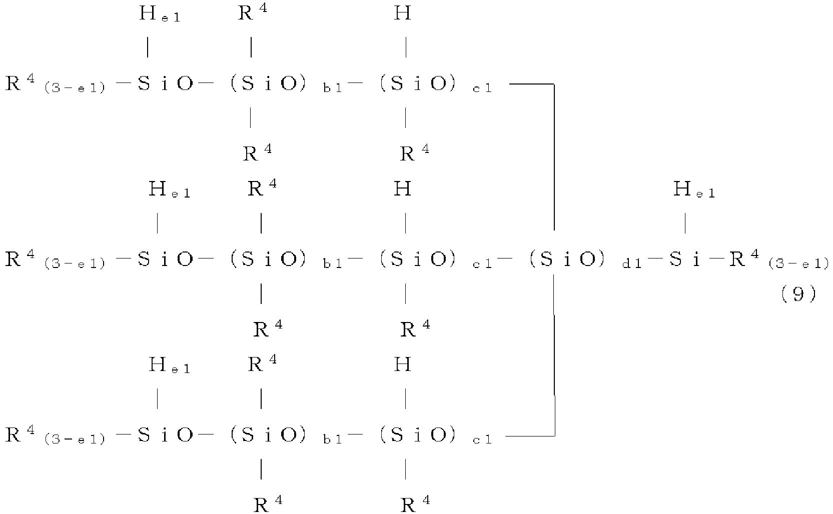 In the formula, R⁴ is the same as above, "b1" is a positive number, "c1" is 0 or a positive number, "d1" is a positive number, and "e1" is 0, 1, 2, or 3, provided that "c1" and "e1" are numbers that satisfy c1+e1≥1.

Further, examples include those represented by the following unit formula (10) that have two or more hydrogen atoms bonded to a silicon atom per molecule.

[R⁴₃SiO_{1/2}]_{f1}[H(R⁴)₂SiO_{1/3}]_{g1}[SiO_{4/2}]ₕ₁[(OR⁵)SiO_{3/2}]ᵢ₁ (10)

In the formula, R⁴ is the same as above, R⁵ is a hydrogen atom or an unsubstituted monovalent hydrocarbon group having 1 to 6 carbon atoms, "f1" is 0 or a positive number, "g1" is a positive number, "h1" is a positive number, and "i1" is 0 or a positive number.

As mentioned above, component (A) is an organopolysiloxane having two or more alkenyl groups in one molecule, and component (B) is an organohydrogenpolysiloxane having two or more hydrogen atoms bonded to a silicon atom in one molecule. However, the combination of component (A) organopolysiloxane having only two alkenyl groups and component (B) organohydrogenpolysiloxane having only two SiH groups is excluded. This is because the combination of the organopolysiloxane having two alkenyl groups as component (A) and the organohydrogensiloxane having two SiH groups as component (B) makes their cured product sticky, making it impossible to obtain a silicone rubber. That is, when component (A) has two alkenyl groups, at least one of components (B) is an organohydrogensiloxane having three or more SiH groups, and when component (B) has two SiH groups, at least one of components (A) is an organosiloxane having three or more alkenyl groups.

The amount of component (B) relative to component (A) is preferably an amount such that the ratio of the number of SiH groups in component (B) relative to the number of monovalent alkenyl groups in component (A) is preferably 0.5 to 2, and more preferably 0.7 to 1.5. When component (B) is blended in an amount such that the ratio of the number of SiH groups falls within the above range, the obtained silicone rubber cured product is not sticky and has appropriate reactivity.

Examples of the catalyst for the addition reaction include platinum group-based metal catalysts used in a hydrosilylation reaction. Examples thereof include platinum group metal simple substances such as platinum (including platinum black), rhodium, and palladium; platinum chlorides, chloroplatinic acids, and chloroplatinates such as H₂PtCl₄·XH₂O, H₂PtCl₆·XH₂O, NaHPtCl₆·XH₂O, KHPtCl₆·XH₂O, Na₂PtCl₆·XH₂O, K₂PtCl₄·XH₂O, PtCl₄·XH₂O, PtCl₂, and Na₂HPtCl₄·XH₂O (wherein X is an integer of 0 to 6, preferably 0 or 6); alcohol-modified chloroplatinic acid; complexes of platinum chloride or chloroplatinic acid and olefins; complexes of chloroplatinic acid and vinyl group-containing siloxanes, and complexes of platinum and vinyl group-containing siloxanes; platinum group metals, such as platinum black and palladium, supported on carriers such as alumina, silica, and carbon; rhodium-olefin complexes; and chlorotris(triphenylphosphine)rhodium (Wilkinson's catalyst). One kind of these catalysts may be used alone, or two or more kinds thereof may be used in combination.

The platinum group-based metal catalyst may be added in an amount that is effective as a hydrosilylation reaction catalyst, and the amount of platinum group metals in the platinum group-based metal catalyst relative to the combined amount of components (A) and (B) is usually about 0.1 to 500 ppm, preferably about 0.5 to 200 ppm, and more preferably about 1 to 100 ppm, by mass.

The silicone rubber may contain a silicone oil, an organosilane, an inorganic powder, an organic powder, an antioxidant, etc.

In the present invention, the density of the rubber particle adhered to the surface of the organic resin particle is not particularly limited. That is, the rubber particles may be sparsely adhered to the surface of the organic resin particle, or may cover and adhere to the surface of the organic resin particle without gaps. The rubber particles may be adhered in an aggregated state, or may form a structure in which rubber particles are adhered further onto the rubber particles that cover and adhere to the surface of the organic resin particle without gaps. The density and state of adhesion of the rubber particles can be confirmed by an electron microscope.

The amount of the rubber particle is not particularly limited, but when a more pronounced soft feeling to the touch, a smooth feeling in use, spreadability, and light scattering properties are desired, the amount is preferably 0.1 parts by mass or more relative to 100 parts by mass of the organic resin particle, more preferably 0.2 parts by mass or more, further preferably 0.5 parts by mass or more, and even more preferably 0.8 parts by mass or more. In addition, from the viewpoint of obtaining low aggregativity, a smooth feeling in use, and spreadability, the amount is preferably 100 parts by mass or less relative to 100 parts by mass of the organic resin particle, more preferably 50 parts by mass or less, further preferably 20 parts by mass or less, and even more preferably 10 parts by mass or less.

### [Silica]

The silica used in the present invention is a binder between the organic resin particle and the rubber particle. By forming the composite particle in which the rubber particles are adhered to the surface of the organic resin particle using the silica as a binder, the rubber particles are fixed to the surface of the organic resin particle and are difficult to fall off from the surface of the organic resin particle, making it possible to provide a better feeling in use.

The silica may be in the form of film or particles, and may be adhered partially or entirely to the surface of the organic resin particle and/or the surface of the rubber particle.

Silica has a structure consisting of SiO₂ units, and although there are no particular limitations on the method for manufacturing it, the silica is preferably obtained by a hydrolysis and condensation reaction of tetraalkoxysilane, as in the production method described later.

The amount of silica is not particularly limited, but in order to fix the rubber particles to the surface of the organic resin particle, the amount of silica is preferably 10 parts by mass or more, more preferably 20 parts by mass or more, furthermore preferably 30 parts by mass or more, and even further preferably 50 parts by mass or more, relative to 100 parts by mass of the rubber particles. Also, from the viewpoint of obtaining a soft feeling to the touch, the amount is preferably 1000 parts by mass or less, more preferably 800 parts by mass or less, further preferably 500 parts by mass or less, and even further preferably 350 parts by mass or less, relative to 100 parts by mass of the rubber particle.

### [Surface Treatment Agent]

The inventive composite particle may have their surface treated with a silylation agent, a silicone oil, waxes, paraffins, an organic fluorine compound, a surfactant, etc., in order to impart or improve water repellency and improve dispersibility in an oil agent.

### [Method for Manufacturing Composite Particle]

The inventive composite particle formed by adhering rubber particles to the surface of organic resin particle using silica as a binder can be obtained by adding tetraalkoxysilane to a liquid mixture of the organic resin particle, the rubber particle, a cationic substance (cationic surfactant and/or cationic water-soluble polymer), an alkaline substance, and water, and then subjecting the tetraalkoxysilane to a hydrolysis/condensation reaction.

### [Organic Resin Particle and Rubber Particle]

For the organic resin particle and the rubber particle, the same as those explained in the composite particle section are used, but it is also possible to use those prepared as an aqueous dispersion in advance or those synthesized in water as an aqueous dispersion.

In order to disperse the organic resin particle and the rubber particle in water, a surfactant and a water-soluble polymer may be blended into the aqueous dispersion. When an aqueous dispersion for synthesis in water is used, the surfactant and the water-soluble polymer used during the synthesis may be contained in the aqueous dispersion.

The surfactant is not particularly limited, but a nonionic surfactant, a cationic surfactant, and/or an amphoteric surfactant is preferable.

Examples of nonionic surfactants include polyoxyethylene alkyl ethers, polyoxyethylene polyoxypropylene alkyl ethers, polyoxyethylene alkylphenyl ethers, polyethylene glycol fatty acid esters, sorbitan fatty acid esters, polyoxyethylene sorbitan fatty acid esters, polyoxyethylene sorbit fatty acid esters, glycerin fatty acid esters, polyoxyethylene glycerin fatty acid esters, polyglycerin fatty acid esters, propylene glycol fatty acid esters, polyoxyethylene castor oil, polyoxyethylene hydrogenated castor oil, polyoxyethylene hydrogenated castor oil fatty acid esters, polyoxyethylene alkylamine, polyoxyethylene fatty acid amide, polyoxyethylene modified organopolysiloxanes, and polyoxyethylene polyoxypropylene modified organopolysiloxane, etc.

Examples of the cationic surfactant include, but are not limited to, alkyltrimethylammonium salts, dialkyldimethylammonium salts, polyoxyethylenealkyldimethylammonium salts, dipolyoxyethylenealkylmethylammonium salts, tripolyoxyethylenealkylammonium salts, alkylbenzyldimethylammonium salts, alkylpyridinium salts, monoalkylamine salts, and monoalkylamidoamine salts, etc.

Examples of amphoteric surfactants include alkyl dimethylamine oxide, alkyl dimethyl carboxybetaine, alkyl amidopropyl dimethyl carboxybetaine, alkyl hydroxysulfobetaine, and alkyl carboxymethyl hydroxyethyl imidazolinium betaine, etc.

The water-soluble polymer is not particularly limited, but a nonionic water-soluble polymer and/or a cationic water-soluble polymer are preferable.

Examples of the nonionic water-soluble polymer include polyvinyl alcohol, polyacrylamide, polyvinylpyrrolidone, polyethylene oxide, polymethyl vinyl ether, polyisopropylacrylamide, methyl cellulose, hydroxypropyl methyl cellulose, starch, guar gum, and xanthan gum, etc.

Examples of cationic water-soluble polymers include dimethyldiallylammonium chloride polymers, vinylimidazoline polymers, methylvinylimidazolium chloride polymers, ethyltrimethylammonium chloride acrylate polymers, ethyltrimethylammonium chloride methacrylate polymers, acrylamidopropyltrimethylammonium chloride polymers, methacrylamidopropyltrimethylammonium chloride polymers, epichlorohydrin/dimethylamine polymers, ethyleneimine polymers, quaternized ethyleneimine polymers, allylamine hydrochloride polymers, polylysine, cationic starch, cationic cellulose, chitosan, and derivatives thereof obtained by copolymerizing monomers having nonionic or anionic groups with these, etc.

When a silicone rubber particle is used as the rubber particle, it is preferable to use an aqueous dispersion of the silicone rubber particle.

The method for preparing the aqueous dispersion of the silicone rubber particle is not particularly limited, and any known method for preparing an aqueous dispersion of a silicone rubber particle can be used. For example, the curable liquid silicone composition described in the composite particle section can be emulsified in water using the surfactant and/or the water-soluble polymer described above, and then subjected to a curing reaction to manufacture the aqueous dispersion.

When making an aqueous dispersion of the silicone rubber particle by curing due to an addition reaction, the method can include one in which the curable liquid silicone composition including component (A) organopolysiloxane having alkenyl groups and component (B) organohydrogenpolysiloxane having silicon atom-bonded hydrogen atoms described in the composite particle section is emulsified by adding the aforementioned surfactant and/or the aforementioned water-soluble polymer and water, and then added with a platinum catalyst after the emulsification to carry out addition polymerization. The method can also be a method in which a platinum group-based metal catalyst is mixed in advance in the curable liquid silicone composition, but in such a case, it is necessary to prevent the polymerization from proceeding before the emulsification is completed by adjusting the temperature and the amount of catalyst and mixing a control agent, etc.

In the liquid mixture containing the organic resin particle, the rubber particle, the cationic substance, the alkaline substance, and water, the organic resin particle is contained in an amount of preferably 3 to 150 parts by mass, more preferably 5 to 50 parts by mass, relative to 100 parts by mass of the blended water. At 3 parts by mass or more, the manufacturing efficiency is high, and at 150 parts by mass or less, the kinetic viscosity of the aqueous dispersion (liquid mixture) is not too high, making the adhesion of the rubber particles easy. The amount of the rubber particle may be the amount to be adhered to the surface of the organic resin particle.

### [Cationic Substance]

The cationic surfactant and/or cationic water-soluble polymer blended during the manufacture of the composite particle have the effect of promoting the condensation reaction of the hydrolyzed tetraalkoxysilane and generating silica. They are also thought to have the effect of adsorbing the generated silica to the surface of the organic resin particle and the rubber particle, and the effect of adsorbing the rubber particle to the organic resin particle.

One kind of the cationic surfactant and the cationic water-soluble polymer may be used alone, or two or more kinds thereof may be used in combination. It is preferable to use both the cationic surfactant and the cationic water-soluble polymer because they can adhere a large amount of the rubber particle to the surface of the organic resin particle.

Examples of the cationic surfactant include those mentioned above in the description of [Method for Manufacturing Composite Particle], but alkyltrimethylammonium salts are preferable. Among them, a lauryltrimethylammonium salt and a cetyltrimethylammonium salt are more preferable.

Examples of the cationic water-soluble polymer include those mentioned above in the description of [Method for Manufacturing Composite Particle], but among them, polymers that do not use monomers having a nonionic group are preferable, and polymers of dimethyldiallylammonium chloride are more preferable.

The amount of the cationic substance (cationic surfactant and/or cationic water-soluble polymer) is preferably within the range of 0.0001 to 2.0 parts by mass, more preferably 0.001 to 1.0 part by mass, relative to 100 parts by mass of water blended in the liquid mixture. When the blending amount is 0.0001 to 2.0 parts by mass, the rubber particle adheres to the surface of the organic resin particle, and the fixation by silica is sufficient.

Furthermore, it is preferable that the amount of the cationic surfactant is 0.001 to 1.9 parts by mass and the amount of the cationic water-soluble polymer is 0.0001 to 1.0 part by mass relative to 100 parts by mass of water blended in the liquid mixture. It is more preferable that the amount of the cationic surfactant is 0.01 to 1.0 part by mass and the amount of the cationic water-soluble polymer is 0.001 to 0.5 parts by mass. It is further preferable that the amount of the cationic surfactant is 0.05 to 0.5 parts by mass and the amount of the cationic water-soluble polymer is 0.01 to 0.2 parts by mass.

As described above, when a cationic surfactant and/or cationic water-soluble polymer is used in order to disperse the organic resin particle and the rubber particle in water to prepare an aqueous dispersion in advance, if the desired type and amount of cationic surfactant and/or cationic water-soluble polymer is not obtained, then additional adding them is required, but if the desired type and amount are obtained, then the additional addition is not necessary.

The cationic surfactant and/or cationic water-soluble polymer may be blended in advance in a liquid mixture of the organic resin particle, the rubber particle, the alkaline substance, and water, or may be added simultaneously with the addition of the tetraalkoxysilane described below.

### [Alkaline Substance]

The alkaline substance acts as a catalyst to make tetraalkoxysilane undergo a hydrolysis and a condensation reaction.

The alkaline substance is not particularly limited. For example, it is possible to use alkali metal hydroxides such as potassium hydroxide, sodium hydroxide, and lithium hydroxide; alkaline-earth metal hydroxides such as calcium hydroxide and barium hydroxide; alkali metal carbonates such as potassium carbonate and sodium carbonate; ammonia; tetraalkylammonium hydroxides such as tetramethylammonium hydroxide and tetraethylammonium hydroxide; or amines such as monomethylamine, monoethylamine, monopropylamine, monobutylamine, monopenthylamine, dimethylamine, diethylamine, trimethylamine, triethanolamine, and ethylenediamine. Among them, ammonia is the most preferable because ammonia can be removed easily from powder of the obtained composite particle by volatilization. As the ammonia, a commercially available aqueous ammonia solution can be used.

The blending amount of the alkaline substance is preferably an amount at which the pH of the liquid mixture blended at least with the alkaline substance, the organic resin particle, the rubber particle, the cationic surfactant and/or the cationic water-soluble polymer, and water, preferably falls within the range of 9.0 to 12.0, more preferably 9.5 to 11.5. When the alkaline substance is added so that the pH falls within the range of 9.0 to 12.0, the hydrolysis and the condensation reaction of tetraalkoxysilane can proceed sufficiently, and the rubber particle fixes on a surface of the organic resin particle sufficiently.

### [Tetraalkoxysilane]

In the inventive method for manufacturing the composite particle, tetraalkoxysilane is added to a liquid mixture of the organic resin particle, the rubber particle, the cationic surfactant and/or the cationic water-soluble polymer, the alkaline substance, and water.

Tetraalkoxysilane undergoes a hydrolysis and condensation reaction under the catalytic action of the alkaline substance to become silica. After the addition of tetraalkoxysilane, silica generated by the hydrolysis and condensation reaction of tetraalkoxysilane is formed on the surface of the organic resin particle and/or the surface of the rubber particle, and at the same time, the rubber particles are adsorbed to the surface of the organic resin particle, resulting in a state in which the rubber particles are fixed to the surface of the organic resin particle by silica.

The tetraalkoxysilane is represented by Si(OR⁶)₄. In the formula, R⁶ represents an alkyl group. The alkyl group preferably has 1 to 6 carbon atoms. Specific examples include a methyl group, an ethyl group, a propyl group, a butyl group, a pentyl group, a hexyl group, etc., but, in view of reactivity, a methyl group and an ethyl group are more preferable. That is, tetramethoxysilane and tetraethoxysilane are more preferable, and tetramethoxysilane is further preferable. As the tetraalkoxysilane, one in which a part or all of the alkoxy groups have been hydrolyzed may be used. Furthermore, a partially condensed one may be used.

The added amount of tetraalkoxysilane is not particularly limited, and preferably such an amount that the amount of silica is the above-described amount relative to the amount of the rubber particle after the hydrolysis and condensation reaction.

### [Hydrolysis and Condensation Reaction]

The hydrolysis and condensation reaction was carried out, by adding the tetraalkoxysilane to the liquid mixture of the organic resin particle, the rubber particle, the cationic surfactant and/or the cationic water-soluble polymer, the alkaline substance, and water, while stirring the mixture. The tetraalkoxysilane may be added dropwise gradually or may be added in a form dissolved or dispersed in water or may be added in a form blended with a water-soluble organic solvent such as alcohol.

In order to prevent aggregation among the organic resin particles and among the rubber particles, it is preferable to stir gently using a paddle blade, a propeller blade, a swept-back blade, an anchor-shaped blade, etc., but the stirring intensity must be strong enough to disperse the organic resin particle, the rubber particle, and the tetraalkoxysilane in the liquid mixture.

The temperature when the tetraalkoxysilane is added to the liquid mixture is preferably 0 to 60°C, and more preferably 0 to 39°C. When the temperature is 0°C or higher, there is no risk of solidifying the liquid mixture, and when the temperature is 60°C or lower, there is no risk of aggregating the obtained particles.

A water-soluble organic solvent such as alcohol may be added to the liquid mixture in order to improve the dispersibility of the organic resin particle and the rubber particle in water and adhere the rubber particle to the surface of the organic resin particle uniformly.

When the particles are surface-treated with a silylating agent, the finished composite particle may be treated, or the silylating agent may be added after adding tetraalkoxysilane to the liquid mixture for treatment. Examples of the silylating agent include trimethylmethoxysilane, trimethylsilanol, hexamethyldisilazane, methyltrimethoxysilane, phenyltrimethoxysilane, etc.

After the addition of tetraalkoxysilane is completed, it is preferable to continue stirring for a while until the hydrolysis and condensation reaction is completed. In order to promote the reaction, heating at 40 to 100°C or adding an alkaline substance may be performed. After that, if necessary, an acidic substance may be added to neutralize the mixture.

After the hydrolysis/condensation reaction, water is removed. The removal of water may be performed by subjecting the reacted liquid mixture to a heat treatment under normal pressure or under reduced pressure. Specific examples include a method of removing water by leaving the liquid mixture to stand under heating; a method of removing water by agitating and fluidizing the liquid mixture under heating; a method of spraying and dispersing the liquid mixture into such a hot air flow as a spray dryer; a method of using a flow of hot medium; etc. Note that, the liquid mixture may be condensed by methods such as thermal dehydration, filtration separation including pressure filtration, centrifugal separation, and decantation as a pre-treatment of this operation. If necessary, the liquid mixture may be washed with water, alcohol, or the like.

When the powder obtained by removing water from the reactioned liquid mixture has aggregated, the aggregates may be disintegrated or classified using a pulverizer such as a jet mill, a ball mill, and a hammer mill.

### [Cosmetic containing Composite Particle]

The inventive cosmetic contains the composite particle. Hereinafter, the inventive cosmetics is described in detail. Note that, in the present invention, component names may be written according to Labeling Name of Cosmetic Ingredient or International Nomenclature of Cosmetic Ingredient (INCI). Regarding components having Japanese Labeling Name corresponding to that of INCI, the English description of the Labeling Name may be omitted.

The present invention may be applicable to various cosmetics, but is particularly preferably applied to cosmetics for external use on the skin, such as skin care cosmetics, makeup cosmetics, antiperspirant cosmetics, and ultraviolet light-protecting cosmetics, and cosmetics for external use on hair such as hair cosmetics. Examples of the skin care cosmetics include skin lotion, milky lotion, cream, cleansing, pack, oil liquid, massaging agent, beauty essence, beauty oil, cleaning agent, deodorant, hand creams, lip creams, and wrinkle concealment. Examples of the makeup cosmetics include make-up base, concealer, white powder, powder foundation, eye color, eye shadow, mascara, eyeliner, eyebrow, and lipstick. Examples of the antiperspirant cosmetics include a roll-on type, a cream type, a solution type, a stick type, etc. Examples of the ultraviolet light-protecting cosmetics include sunscreen oil, sunscreen emulsion, sunscreen cream, etc. Examples of hair cosmetics include shampoo, conditioner, treatment, setting agents, etc.

The inventive cosmetic may be in any forms including powder, oil-based liquids, water-in-oil emulsions, oil-in-water emulsions, non-aqueous emulsions, multiple emulsions such as W/O/W and O/W/O, etc., for example. Further, the forms of the inventive cosmetic can be selected from a variety of forms such as liquid, emulsion, cream, solid, paste, gel, powder, pressed, multilayer, mousse, spray, stick, and pencil.

The cosmetic is not particularly limited as long as containing the essential components, but can be applied for skin care cosmetics, liquid foundation, powder foundation, concealer, lipstick, a variety of products in which sunscreen function imparted, etc.

The inventive cosmetic can contain a variety of components used in general cosmetics. The inventive cosmetic may contain, for example, (1) an oil agent, (2) a water-based component, (3) a surfactant, (4) powder other than those of the present invention, (5) a composition including a crosslinked organopolysiloxane and an oil agent in a liquid state at room temperature, (6) a film-forming agent, (7) an ultraviolet absorbing-scattering agent, and (8) other additives. One kind of thereof can be used alone, or two or more kinds thereof may be used in appropriate combination.

### (1) Oil agent

The oil agent may be volatile or nonvolatile, and may be any state among solid, semi-solid, and liquid, at room temperature (25°C). The examples thereof include silicone oils, silicone waxes, natural animal and vegetable oils and fats, semi-synthetic oils and fats, hydrocarbon oils, higher alcohols, fatty acids, ester oils, fluorinated oils, ultraviolet absorbers, etc.

The inventive composite particle has a high oil-absorbing property for various liquid oils due to the rubber particles on the surface, and by absorbing the liquid oil, the sticky feeling and glitter of the oil can be reduced, and the effect of improving a feeling in use is high. In addition, the viscosity of the oil can be adjusted, and various properties such as a mousse-like state can be obtained. The composite particle used in the present invention can be directly contained as a cosmetic ingredient, but for the purpose of producing a desired feeling in use of the cosmetic, an oily gel composition containing the composite particle and the oil-based component may be separately prepared, and then the cosmetic containing the composite particle and the oil-based component may be prepared in the form of the oily gel composition.

### · Silicone oil

Examples of the silicone oil include alkyl-modified silicones such as Dimeticone (INCI), Trisiloxane (INCI), Methyl Trimethicone (INCI), Ethyl Trisiloxane (INCI), Ethyl Methicone (INCI), and Hexyl Dimethicone (INCI); long-chain alkyl-modified silicones such as Caprylyl Methicone (INCI); linear or branched organopolysiloxanes having low to high viscosity such as Phenyl Trimethicone (INCI), Diphenyl Dimethicone (INCI), Diphenylsiloxy Phenyl Trimethicone (INCI), Tetraphenyl Dimethyl Disiloxane (INCI), and Methylhydrogenpolysiloxane; cyclic organopolysiloxanes such as Cyclotetrasiloxane(INCI), Cyclopentasiloxane(INCI), Cyclohexasiloxane(INCI); amino-modified organopolysiloxanes such as Amodimethicone (INCI) and Aminopropyl Dimethicone (INCI); pyrrolidone-modified organopolysiloxanes such as PCA Dimethicone (INCI); pyrrolidone carboxylic acid-modified organopolysiloxane; silicone rubbers such as gum-like dimethylpolysiloxane having a high degree of polymerization, gum-like amino-modified organopolysiloxane, and a gum-like dimethylsiloxane-methylphenylsiloxane copolymer; low-viscosity organopolysiloxane solutions of silicone gum or rubber; and dissolved products of amino acid-modified silicones, fluorine-modified silicones, and silicone resins; etc.

Examples of the commercial products of silicone oil include those manufactured by Shin-Etsu Chemical Co.: KF-96L-1cs, KF-96L-1.5cs, KF-96L-2cs, KF-96A-6cs, KF-4422, KF-54, KF-54HV, KF-56A, KF-995, etc.

### Oil-Based Component in Solid State

In the present invention, when the cosmetic is to be hardened, the cosmetic preferably contains an oil-based component being solid at 25°C. The oil-based component being solid at 25°C preferably have a melting point of preferably 40°C or higher, more preferably 60 to 110°C. Such oil-based components can include waxes, hydrocarbons, esters, higher alcohols, and higher fatty acids, and are not particularly limited as long as it is a raw material which can be commonly contained into cosmetics.

Specific examples thereof include vegetable waxes such as carnauba Wax (INCI: Copernicia Cerifera (Carnauba) Wax), sugar cane wax, candelilla Wax (INCI: Euphorbia Cerifera (Candelilla) Wax), purified candelilla wax, rice wax, japan wax, jojoba wax, kapok wax, rice bran wax, myrica cerifera fruit wax, shea butter, cacao butter, rice bran (INCI: Rhus Succedanea Fruit Wax), montan wax (INCI: Montan Wax), and hydrogenated castor oil isostearate; animal waxes such as beeswax, tallow, neat's bone fat, lard (INCI: Lard), horse fat (INCI: Horse Fat), mutton tallow, lanolin (INCI: Lanolin), chuhokuro, shellac wax, and spermaceti; semisynthetic oil such as lanolin ester, lanolin fatty acid ester, and beeswax acid ester; hardened oil such as hardened castor oil, and hardened coconut oil; hydrocarbon-based waxes such as solid paraffin, polyethylene, ceresin, ozokerite, and microcrystalline wax; wax esters such as synthetic beeswax; amino acid stearyl alcohol such as dioctyldodecyl lauroyl glutamate, dioctyldodecyl lauroyl glutamate, and dioctyldodecyl lauroyl glutamate; fatty acids such as stearic acid and behenic acid; and silicone waxes such as acrylic-silicone resins that are acrylic-silicone graft or block copolymers (acrylic-silicone graft copolymers: KP-561P, 562P, etc., manufactured by Shin-Etsu Chemical Co., Ltd.), or derivatives thereof. It is preferable that the oil-based component is one kind or two or more kinds selected from these.

### · Natural animal and vegetable oil agents and semi-synthetic oil agents

Natural animal and vegetable oil agents and semi-synthetic oil agents include: germ oils such as avocado oil (: Labeling Name (INCI: Persea Gratissima (Avocado) Oil)), linseed oil (: Labeling Name (INCI: Linum Usitatissimum (Linseed) Seed Oil)), almond oil (: Labeling Name (INCI: Prunus Amygdalus Dulcis (Sweet Almond) Oil)), perilla oil (Labeling Name), olive oil (: Labeling Name (INCI: Olea Europaea (Olive) Fruit Oil)), Torreya Californica oil (: Labeling Name (INCI: Torreya Californica (California Nutmeg) Oil)), Cymbopogon Nardus oil (: Labeling Name (INCI: Cymbopogon Nardus (Citronella) Oil)), Torreya Nucifera seed oil (: Labeling Name (INCI: Torreya Nucifera Seed Oil)), Kyounin oil (: Labeling Name (INCI: Kyounin Yu)), wheat germ oil (: Labeling Name (INCI: Triticum Vulgare (Wheat) Germ **Oil)),** sesame oil (: Labeling Name (INCI: Sesamum Indicum (Sesame) Seed **Oil)),** wheat germ oil (: Labeling Name (INCI: Triticum Vulgare (Wheat) Germ Oil)), rice germ oil (: Labeling Name (INCI: Oryza Sativa (Rice) Germ **Oil)),** rice bran oil (: Labeling Name (INCI: Oryza Sativa (Rice) Bran **Oil)),** sasanqua oil (: Labeling Name (INCI: Camellia Kissi Seed Oil)), safflower oil (: Labeling Name (INCI: Carthamus Tinctorius (Safflower) Seed **Oil)),** Soybean oil (: Labeling Name (INCI: Glycine Soja (Soybean) Oil)), camellia sinensis seed oil (: Labeling Name (INCI: Camellia Sinensis Seed Oil)), camellia oil (: Labeling Name (INCI: Camellia Japonica Seed Oil)), evening primrose oil (: Labeling Name (INCI: Oenothera Biennis (Evening Primrose) Oil)), rape seed oil (Labeling Name), corn germ oil (: Labeling Name (INCI: Zea Mays (Corn) Germ Oil)), and wheat germ oil (: Labeling Name (INCI: Triticum Vulgare (Wheat) Germ Oil)); natural vegetable oil such as persic oil (Labeling Name), palm oil (: Labeling Name (INCI: Elaeis Guineensis (Palm) Oil)), palm kernel oil (: Labeling Name (INCI: Elaeis Guineensis (Palm) Kernel Oil)), castor oil (: Labeling Name (INCI: Ricinus Communis (Castor) Seed Oil)), sunflower oil (: Labeling Name (INCI: Helianthus Annuus (Sunflower) Seed Oil)), grape seed oil (: Labeling Name (INCI: Vitis Vinifera (Grape) Seed Oil)), Jojoba seed oil (: Labeling Name (INCI: Simmondsia Chinensis (Jojoba) Seed Oil)), Macadamia seed oil (: Labeling Name (INCI: Macadamia Ternifolia Seed Oil)), Meadowfoam oil (: Labeling Name (INCI: Limnanthes Alba (Meadowfoam) Seed Oil)), cotton seed oil (: Labeling Name (INCI: Gossypium Herbaceum (Cotton) Seed Oil)), coconut oil (: Labeling Name (INCI: Cocos Nucifera (Coconut) Oil)), and peanut oil (: Labeling Name (INCI: Arachis Hypogaea (Peanut) Oil)); natural animal oil such as shark liver oil (: Labeling Name (INCI: Shark Liver Oil)), cod liver oil (: Labeling Name (INCI: Cod Liver Oil)), fish liver oil (: Labeling Name (INCI: Fish Liver Oil)), turtle oil (: Labeling Name (INCI: Turtle Oil)), mink oil (: Labeling Name (INCI: Mink Oil)), and egg oil (: Labeling Name (INCI: Egg Oil)); and semi-synthetic oil and fat such as hydrogenated coconut oil (: Labeling Name (INCI: Hydrogenated Coconut Oil)) and liquid lanolin (: Labeling Name (INCI: Lanolin Oil)).

### Hydrocarbon oil

Examples of the hydrocarbon oil include linear or branched hydrocarbon oils. The hydrocarbon oil may be volatile hydrocarbon oil or nonvolatile hydrocarbon oil. Specific examples thereof include isoparaffins such as Olefin Oligomer (INCI) and (C13, 14) Isoparaffin (INCI); and alkanes such as Isododecane (INCI), Undecane (INCI), Dodecane (INCI), Isohexadecane (INCI), Hydrogenated Polyisobutene (: Labeling Name (INCI: Hydrogenated Polyisobutene)), Squalane (INCI), Mineral Oil (INCI), Coconut Alkanes (INCI), and (C13-15) Alkane (INCI).

### · Higher alcohol

Examples of the higher alcohols include alcohols having preferably 6 or more carbon atoms, more preferably 10 to 30 carbon atoms. Specific example of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyldodecanol (INCI), Cholesterol (INCI), Phytosterols (INCI), Batyl Alcohol (INCI), etc.

### Ester oils

Examples of the ester oils include: diisobutyl adipate (: Labeling Name (INCI: Diisobutyl Adipate)), dihexyldecyl adipate (Labeling Name), diheptylundecyl adipate (: Labeling Name (INCI: Diheptylundecyl Adipate)), n-alkylglycol monoisostearate such as isostearyl isostearate (: Labeling Name (INCI: Isostearyl Isostearate)), isocetyl isostearate (: Labeling Name (INCI: Isocetyl Isostearate)), trimethylolpropane triisostearate (: Labeling Name (INCI: Trimethylolpropane Triisostearate)), glycol diethylhexanoate (: Labeling Name (INCI: Glycol Diethylhexanoate)), cetyl ethylhexanoate (: Labeling Name (INCI: Cetyl Ethylhexanoate)), trimethylolpropane triethylhexanoate (: Labeling Name (INCI: Trimethylolpropane Triethylhexanoate)), pentaerythrityl tetraethylhexanoate (: Labeling Name (INCI: Pentaerythrityl Tetraethylhexanoate)), cetyl octanoate (: Labeling Name (INCI: Cetyl Ethylhexanoate)), octyldodecyl esters such as octyldodecyl stearoyloxy stearate (: Labeling Name (INCI: Octyldodecyl Stearoyl Stearate)), oleyl oleate (: Labeling Name (INCI: Oleyl Oleate)), octyldodecyl oleate (: Labeling Name (INCI: Octyldodecyl Oleate)), decyl oleate (: Labeling Name (INCI: Decyl Oleate)), neopentyl glycol dioctanoate (: Labeling Name (INCI: Neopentyl Glycol Diethylhexanoate)), neopentyl glycol dicaprate (: Labeling Name (INCI: Neopentyl Glycol Dicaprate)), diisostearyl malate (: Labeling Name (INCI: Diisostearyl Malate)), triethyl citrate (: Labeling Name (INCI: Triethyl Citrate)), diethylhexyl succinate (: Labeling Name (INCI: Diethylhexyl Succinate)), amyl acetate (: Labeling Name (INCI: Amyl Acetate)), etyl acetate (: Labeling Name (INCI: Etyl Acetate)), butyl acetate (: Labeling Name (INCI: Butyl Acetate)), isocetyl stearate (: Labeling Name (INCI: Isocetyl Stearate)), butyl stearate (: Labeling Name (INCI: Butyl Stearate)), diisopropyl sebacate (: Labeling Name (INCI: Diisopropyl Sebacate)), diethylhexyl sebacate (: Labeling Name (INCI: Diethylhexyl Sebacate)), cetyl lactate (: Labeling Name (INCI: Cetyl Lactate)), myristyl lactate (: Labeling Name (INCI: Myristyl Lactate)), isononyl isononanoate (: Labeling Name (INCI: Isononyl Isononanoate)), isotridecyl isononanoate (: Labeling Name (INCI: Isotridecyl Isononanoate)), palmitic acid esters such as isopropyl palmitate (: Labeling Name (INCI: Isopropyl Palmitate)), ethylhexyl palmitate (: Labeling Name (INCI: Ethylhexyl Isopalmitate)), and hexyldecyl palmitate (: Labeling Name (INCI: Isocetyl Palmitate, Hexyldecyl Palmitate)), cholesteryl hydroxystearate (: Labeling Name (INCI: Cholesteryl Hydroxystearate)), myristic acid esters such as isopropyl myristate (: Labeling Name (INCI: Isopropyl Myristate)), octyldodecyl myristate (: Labeling Name (INCI: Octyldodecyl Myristate)), and myristyl myristate (: Labeling Name (INCI: Myristyl Myristate)), ethylhexyl laurate (: Labeling Name (INCI: Ethylhexyl Laurate)), hexyl laurate (: Labeling Name (INCI: Hexyl Laurate)), dioctyldodecyl lauroyl glutamate (: Labeling Name (INCI: Dioctyldodecyl Lauroyl Glutamate)), isopropyl lauroyl sarcosinate (: Labeling Name (INCI: Isopropyl Lauroyl Sarcosinate)), and (caprylate/caprate) coco-alkyl (: Labeling Name (INCI: Coco-Caprylate.Caprate)).

Among the ester oils, examples of glyceride oil include Triethylhexanoin (INCI), Tri (Caprylic/Capric) glyceride (: Labeling Name (INCI: Caprylic/Capric Triglyceride)), Coco-Glycerides (INCI), (Caprylic/Capric/Succinic) Triglyceride (: Labeling Name (INCI: Caprylic/Capric/Succinic Triglyceride)), (Caprylic/Capric) Glycerides (: Labeling Name (INCI: Caprylic/Capric Glycerides)), etc.

### Fluorine-based oil agent

Examples of the fluorine-based oil agent include Perfluorodecalin (INCI), Perfluorononyl Dimethicone (INCI), and Perfluoromethylcyclopentane (INCI), etc.

### · Ultraviolet absorber

Examples of the ultraviolet absorber include oxybenzone-1 (: Labeling Name (INCI: Benzophenone-1)), oxybenzone-2 (: Labeling Name (INCI: Benzophenone-2)), oxybenzone-3 (: Labeling Name (INCI: Benzophenone-3)), oxybenzone-4 (: Labeling Name (INCI: Benzophenone-4)), oxybenzone-5 (: Labeling Name (INCI: Benzophenone-5)), oxybenzone-6 (: Labeling Name (INCI: Benzophenone-6)), oxybenzone-9 (: Labeling Name (INCI: Benzophenone-9)), Homosalate (INCI), Octocrylene (INCI), t-butyl methoxydibenzoylmethane (: Labeling Name (INCI: Butyl Methoxydibenzoylmethane)), ethylhexyl salicylate (: Labeling Name (INCI: Ethylhexyl Salicylate)), diethylamino hydroxybenzoyl hexyl benzoate (: Labeling Name (INCI: Diethylamino Hydroxybenzoyl Hexyl Benzoate)), Polysilicone-15 (INCI), octyl dimethoxybenzylidene dioxoimidazolidine propionate (: Labeling Name (INCI: Ethylhexyl Dimethoxybenzylidene Dioxoimidazolidine Propionate)), terephthalylidene dicamphor sulfonic acid (: Labeling Name (INCI: Terephthalylidene Dicamphor Sulfonic Acid)), Ethylhexyl Triazone (INCI), trimethoxycinnamate methyl bis (trimethylsiloxy) silylisopentyl (: Labeling Name (INCI: Isopentyl Trimethoxycinnamate Trisiloxane)), Drometrizole Trisiloxane (INCI), ethylhexyl dimethyl PABA (: Labeling Name (INCI: Ethylhexyl Dimethyl PABA)), paramethoxy cinnamate isopropyl (: Labeling Name (INCI: Isopropyl Methoxycinnamate)), ethylhexyl methoxycinnamate (: Labeling Name (INCI: Ethylhexyl Methoxycinnamate)), Bis-Ethylhexyloxyphenol Methoxyphenyl Triazine (INCI), phenylbenzimidazole sulfonic acid (: Labeling Name (INCI: Phenylbenzimidazole Sulfonic Acid)), Methylene Bis-Benzotriazolyl Tetramethylbutylphenol (INCI), glyceryl ethylhexanoate dimethoxycinnamate (: Labeling Name (INCI: Glyceryl Ethylhexanoate Dimethoxycinnamate)), Glyceryl PABA (INCI), diisopropyl methyl cinnamate (: Labeling Name (INCI: Diisopropyl Methyl Cinnamate)), Cinoxate (INCI), ethylhexyl dimethoxybenzylidene dioxoimidazolidine propionate (: Labeling Name (INCI: Ethylhexyl Dimethoxybenzylidene Dioxoimidazolidine Propionate)).

### (2) Water-based component

The water-based component is not particularly limited as long as being able to be normally incorporated into cosmetics. Specific examples include water, lower alcohols preferably having 2 to 5 carbon atoms such as ethanol (: Labeling Name (INCI: Alcohol)) and isopropanol (: Labeling Name (INCI: Isopropyl Alcohol)), and sugar alcohols such as Sorbitol (INCI), Maltose (INCI), and Xylitol (INCI). Further, the examples include polyhydric alcohol such as BG (: Labeling Name (INCI: Butylene Glycol)), PG (: Labeling Name (INCI: Propylene Glycol)), DPG (: Labeling Name (INCI: Dipropylene Glycol)), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizing agents such as Glucose (INCI), Glyceryl Glucoside (INCI), Betaine (INCI), Na chondroitin sulfate (: Labeling Name (INCI: Sodium Chondroitin Sulfate)), PCA-Na (: Labeling Name (INCI: Sodium PCA)), Methyl Gluceth-10 (INCI), Methyl Gluceth-20 (INCI), hyaluronic acid, egg yolk lecithin, soybean lecithin, phosphatidylcholine, phosphatidylethanolamine, phosphatidyl serine, phosphatidylglycerol, phosphatidylinositol, sphingophospholipid, etc.

### (3) Surfactant

The surfactant includes nonionic, anionic, cationic, and amphoteric surfactants, but is not particularly limited, and any surfactant can be used as long as it is used for a general cosmetic. Among these surfactants, it is preferable to use one or two or more kinds selected from non-crosslinked or crosslinked silicone surfactants because a stable cosmetic can be obtained. In any case, the content of the surfactant is preferably 0.1 to 20% by mass of the whole cosmetic. When the amount is 0.1% or more, functions such as dispersion and emulsification can be sufficiently achieved. When the amount is 20% by mass or less, there is no risk that the cosmetic has a sticky feeling to the touch in use, and thus is preferable. The HLB of the surfactant is not limited and is preferably 2 to 14.5 for the purpose of maintaining the water resistance of the cosmetic.

In the non-crosslinked silicone surfactants, a part of methyl group of a linear or branched silicone main chain is substituted with a hydrophilic group such as polyethylene glycol, polyglycerin, etc. Specifically, the non-crosslinked silicone surfactant is preferably linear or branched polyoxyethylene-modified organopolysiloxane, linear or branched polyoxyethylene-polyoxypropylene-modified organopolysiloxane, linear or branched polyoxyethylene-alkyl-co-modified organopolysiloxane, linear or branched polyoxyethylene-polyoxypropylene-alkyl-co-modified organopolysiloxane, linear or branched polyglycerin-modified organopolysiloxane, linear or branched polyglycerin-alkyl-co-modified organopolysiloxane, or linear or branched pyrrolidone-modified organopolysiloxane.

Specific examples thereof include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), etc.

Examples of commercial products thereof include those manufactured by Shin-Etsu Chemical Co.: KF-6011, KF-6011P, KF-6012, KF-6015, KF-6017, KF-6043, KF-6028, KF-6038, KF-6048, KF-6100, KF-6104, KF-6106, KF-6105, KF-6115, etc.

Examples of the crosslinked silicone surfactant include (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), (PEG-15/Lauryl Polydimethylsiloxyethyl Dimethicone) Crosspolymer (INCI), (Dimethicone/Polyglycerin-3) Crosspolymer (INCI), (Lauryl Dimethicone/Polyglycerin-3) Crosspolymer (INCI), (Polyglycerin-3/Lauryl Polydimethylsiloxyethyl Dimethicone) Crosspolymer (INCI), etc.

Further, when the crosslinked silicone surfactant is used, in a composition containing the crosslinked silicone surfactant and an oil agent being liquid at room temperature, the crosslinked silicone surfactant is preferably swelled by containing the liquid oil agent in an amount of its own weight or more.

For the liquid oil agent, liquid silicones, hydrocarbon oils, ester oils, natural animal or vegetable oils, semisynthetic oils, or fluorine-based oils, which may belong to in the component (1) oil agent as an antioxidant, can be used. Examples thereof include Cyclopentasiloxane (INCI), Dimethicone (INCI), Mineral Oil (INCI), Isododecane (INCI), Isohexadecane (INCI), Triethylhexanoin (INCI), Isotridecyl Isononanoate (INCI), Squalane (INCI), etc.

Examples of the crosslinked silicone surfactants that are commercial products and swells by incorporating a liquid oil agent include those manufactured by Shin-Etsu Chemical Co.: KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, KSG-850Z, etc.

### (4) Powder

Examples of the powder include color pigment, inorganic powder, metal powder, organic powder, inorganic-organic composite powder, etc. Specific examples thereof are as follows.

### Color pigment

The color pigment is not particularly limited as long as it is a pigment usually used for colorization of cosmetic. It is possible to use any color pigment including: red iron oxide (: Labeling Name (INCI: Iron Oxides)); yellow iron oxide (: Labeling Name (INCI: Iron Oxides)); white titanium dioxide (: Labeling Name (INCI: Titanium Dioxide)); black iron oxide (: Labeling Name (INCI: Iron Oxides)); ultramarines (: Labeling Name (INCI: Ultramarines)); ferric ferrocyanide (: Labeling Name (INCI: Ferric Ferrocyanide, Ferric Ammonium Ferrocyanide)); manganese violet (: Labeling Name (INCI: Manganese Violet)); cobalt titanate (: Labeling Name (INCI: Cobalt Titanium Oxide)); chromium hydroxide (: Labeling Name (INCI: Chromium Hydroxide Green)); chromium oxide (: Labeling Name (INCI: Chromium Oxide Greens)); (Al/cobalt) oxide (: Labeling Name (INCI: Cobalt Aluminum Oxide)); cobalt titanate (: Labeling Name (INCI: Cobalt Titanium Oxide)); (titanium/titanium oxide) burned product (: Labeling Name (INCI: Titanium/Titanium Dioxide)); (Li/cobalt) titanate (: Labeling Name (INCI: Lithium Cobalt Titanate)); cobalt titanate (: Labeling Name (INCI: Cobalt Titanium Oxide)); (iron oxide/titanium oxido) burned product (:Labeling Name); composites doped with different metals such as iron oxide-doped titanium oxide (: Labeling Name (INCI: Iron Oxides, Titanium Dioxide)); titanium nitride (: Labeling Name (INCI: Titanium Nitride)), ferrous hydroxide (: Labeling Name (INCI: Iron Hydroxide)), an inorganic brown pigment such as γ-iron oxide; an inorganic yellow pigment such as yellow ocher, colored pigments such as a laked tar-based dye and a laked natural dye.

Moreover, the pigment may have any shape such as spherical, nearly spherical, rod-like, spindle, petaloid, strip, and irregular forms. The geometrical aspect of the pigment is not particularly limited as long as it can impart color to a cosmetic.

### · Inorganic powder

Examples of the inorganic powder include a fine particle made of zirconium oxide (: Labeling Name (INCI: Zirconium Dioxide)), zinc oxide (: Labeling Name (INCI: Zinc Oxide)), cerium oxide (: Labeling Name (INCI: Cerium Oxide)), Mg oxide (: Labeling Name (INCI: Magnesium Oxide)), Ba sulfate (: Labeling Name (INCI: Barium Sulfate)), calcium sulfate (: Labeling Name (INCI: Calcium Carbonate)), Mg Sulfate (: Labeling Name (INCI: Magnesium Sulfate)), Ca Carbonate (: Labeling Name (INCI: Calcium Carbonate)), Mg Carbonate (: Labeling Name (INCI: Magnesium Carbonate)), Talc (INCI), Mica (INCI), Kaolin (INCI), synthetic fluorphlogopite (: Labeling Name (INCI: Synthetic Fluorphlogopite)), synthetic phlogopite iron (Labeling Name), biotite (: Labeling Name (INCI: Biotite)), K silicate (: Labeling Name (INCI: Potassium Silicate)), Silica (INCI), Al silicate (: Labeling Name (INCI: Aluminum Silicate)), Mg silicate (: Labeling Name (INCI: Magnesium Silicate)), silicic acid (Al/Mg) (: Labeling Name (INCI: Magnesium Aluminum Silicate)), Ca silicate (: Labeling Name (INCI: Calcium Silicate)), silicic acid (Al/Ca/Na) (: Labeling Name (INCI: Aluminum Calcium Sodium Silicate)), silicic acid (Li/Mg/Na) (: Labeling Name (INCI: Lithium Magnesium Sodium Silicate)), silicic acid (Na/Mg) (: Labeling Name (INCI: Sodium Magnesium Silicate)), borosilicate (Ca/Al) (: Labeling Name (INCI: Calcium Aluminum Borosilicate)), borosilicate (Ca/Na) (: Labeling Name (INCI: Calcium Sodium Borosilicate)), Hydroxyapatite (INCI), Bentonite (INCI), Montmorillonite (INCI), Hectorite (INCI), zeolite (INCI), Alumina (INCI), Al hydroxide (: Labeling Name (INCI: Aluminum Hydroxide)), boron nitride (: Labeling Name (INCI: Boron Nitride)), glass (: Labeling Name (INCI: Glass)), and the like.

Moreover, examples of the inorganic coloring pearl pigment include: pearlescent agents such as Mica (INCI) coated with titanium oxide (: Labeling Name (INCI: Titanium Dioxide)) and synthetic fluorphlogopite (: Labeling Name (INCI: Synthetic Fluorphlogopite)) coated with titanium oxide (: Labeling Name (INCI: Titanium Dioxide)); pearl pigments such as bismuth oxychloride (: Labeling Name (INCI: Bismuth Oxychloride)), bismuth oxychloride (: Labeling Name (INCI: Bismuth Oxychloride)) coated with titanium oxide (: Labeling Name (INCI: Titanium Dioxide)), Talc (INCI) coated with titanium oxide (: Labeling Name (INCI: Titanium Dioxide)), fish scale guanine (Labeling Name), and colored mica coated with titanium oxide (: Labeling Name (INCI: Titanium Dioxide)). The inorganic coloring pearl pigment is not particularly limited whether the pigment is non-treated or surface-treated by a known method generally used for cosmetics.

### · Metal powder

Examples of the metal powder include metal fine particles made of Al (: Labeling Name (INCI: Aluminum, Aluminum Powder)), copper (: Labeling Name (INCI: Copper Powder)), silver (: Labeling Name (INCI: Silver Powder)), gold (: Labeling Name (INCI: Gold)), and etc.

### · Organic powder

Examples of the organic powder include powder made of: silicone, polyamide, polyacrylic acid-acrylic acid ester, polyester, Polyethylene (INCI), Polypropylene (INCI), Polystyrene (INCI), styrene-acrylic acid copolymer, divinylbenzene-styrene copolymer, polyurethane, vinyl resin, urea resin, melamine resin, benzoguanamine, polymethylbenzoguanamine, tetrafluoroethylene, polymethyl methacrylate, Cellulose (INCI), Silk (INCI), nylon (Labeling Name), phenol resin, epoxy resin, polycarbonate, or the like.

In particular, examples of the silicone include: silicone resin particles; Polymethylsilsesquioxane (INCI), silicone rubber powder, silicone rubber powder coated with silicone resin; (Vinyl Dimethicone/Methicone Silsesquioxane) Crosspolymer (INCI), (Diphenyl Dimethicone/Vinyl Diphenyl Dimethicone/Silsesquioxane) Crosspolymer (INCI), Polysilicone-1 Crosspolymer (INCI), Polysilicone-22 (INCI); etc.

Examples of commercial products of silicone powder include those manufactured by Shin-Etsu Chemical Co.: KMP-590, KMP-591, KMP-592, KMP-597, KMP-598, KSP-100, KSP-101, KSP-102, KSP-105, KSP-300, KSP-411, KSP-441, KM-9729, KM-440, etc.

Further, examples thereof include metal soap and the like. Specific examples thereof include powder made of zinc stearate (: Labeling Name (INCI: Zinc Stearate)), Al stearate (: Labeling Name (INCI: Aluminum Stearate)), Ca stearate (: Labeling Name (INCI: Calcium Stearate)), Mg stearate (: Labeling Name (INCI: Magnesium Stearate), zinc myristate (: Labeling Name (INCI: Zinc Myristate)), Mg Myristate (: Labeling Name (INCI: Magnesium Myristate)), cetyl phosphate (Zinc/Na) (: Labeling Name (INCI: Sodium Zinc Cetyl Phosphate)), K cetyl phosphate (: Labeling Name (INCI: Potassium Cetyl Phosphatezinc), or the like.

Further, examples of the organic powder include organic dye and the like. Specific examples thereof include tar-based dyes such as Red No. 3, Red No. 104 (1) (: Labeling Name (INCI: Red 28, Red 28 Lake)), Red No. 106, Red No. 201 (: Labeling Name (INCI: Red 6)), Red No. 202 (: Labeling Name (INCI: Red 7)), Red No. 204, Red No. 205, Red No. 220 (: Labeling Name (INCI: Red 34)), Red No. 226 (: Labeling Name (INCI: Red 30)), Red No. 227 (: Labeling Name (INCI: Red 33, RED 33 Lake)), Red No. 228 (: Labeling Name (INCI: Red 36)), Red No. 230 (1) (: Labeling Name (INCI: Red 22, Red 22 Lake)), Red No. 230 (2) (Labeling Name), Red No. 401 (Labeling Name), Red No. 505 (Labeling Name), Yellow No. 4 (: Labeling Name (INCI: Yellow 5)), Yellow No. 5 (: Labeling Name (INCI: Yellow 6, Yellow 6 Lake)), Yellow No. 202 (1) (: Labeling Name (INCI: Yellow 8)), Yellow No. 203 (: Labeling Name (INCI: Yellow 10, Yellow 10 Lake)), Yellow No. 204 (: Labeling Name (INCI: Yellow 11)), Yellow No. 401, Blue No. 1 (: Labeling Name (INCI: Blue 1, Blue 1 Lake)), Blue No. 2, Blue No. 201, Blue No. 205 (: Labeling Name (INCI: Blue 4)), Blue No. 404 (Labeling Name), Green No. 3 (: Labeling Name (INCI: Green 3, Green 3 Lake)), Green No. 201 (: Labeling Name (INCI: Green 5)), Green No. 202 (: Labeling Name (INCI: Green 6)), Green No. 204 (: Labeling Name (INCI: Green 8)), Green No. 205 (Labeling name), Orange No. 201 (: Labeling Name (INCI: Orange 5)), Orange No. 203 (: Labeling Name (INCI: Pigment Orange 5)), Orange No. 204 (Labeling Name), Orange No. 205 (: Labeling Name (INCI: Orange 4, Orange 4 Lake)), Orange No. 206 (: Labeling Name (INCI: Orange 10)), and Orange No. 207 (: Labeling Name (INCI: Orange 11)); and natural dyes such as Cochineal (INCI), laccaic acid (: Labeling Name (INCI: Laccaic Acid)), safflower red (: Labeling Name (INCI: Carthamus Tinctorius (Safflower) Flower Extract)), lithospermum officinale root extract (: Labeling Name (INCI: Lithospermum Officinale Root Extract)), Gardenia florida yellow (Labeling name), and Gardenia florida blue (: Labeling Name (INCI: Hydrolyzed Gardenia Florida Extract)).

### · Inorganic-organic composite powder

Examples of the inorganic-organic composite powder include composite powder in which the surface of inorganic powder is coated with organic powder by a publicly-known general method.

Note that, the above-described powders can be used also after treating their surface. From the viewpoint of water resistance of the cosmetic, the surface treatment agent is preferably capable of imparting hydrophobicity. Such a treatment agent to impart hydrophobicity is not particularly limited, examples thereof include silicone treatment agents; waxes; paraffins; organofluorine compounds of perfluoroalkyl and phosphate or the like; surfactants; amino acids such as N-acyl glutamic acid; metal soaps such as aluminum stearate and magnesium myristate; etc.

The silicone treatment agent is more preferable, and examples thereof include silylating agent or silanes such as triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethyl Hexyl Dimethicone (INCI), (acrylates/tridecyl acrylate/triethoxysilylpropyl methacrylate/dimethicone methacrylate) copolymer (: Labeling Name (INCI: Acrylates/Tridecyl Acrylate/Triethoxysilylpropyl Methacrylate/Dimethicone Methacrylate Copolymer)), etc.

Specific examples of the silicone treatment agent include those manufactured by Shin-Etsu Chemical Co. Ltd.: AES-3083, KF-99P, KF-9901, KF-9908, KF-9909, KP-574, KP-541, etc.

Further, one kind of these surface-hydrophobizing treatment agents may be used alone, or two or more kinds thereof may be used in combination.

Specific examples of a surface-treated color pigment include KTP-09 series manufactured by Shin-Etsu Chemical Co., Ltd., especially KTP-09W, 09R, 09Y, 09B, etc.

### (5) Composition including crosslinked organopolysiloxane and oil agent in liquid state at room temperature

In the composition including a crosslinked organopolysiloxane and an oil agent in a liquid state at room temperature, the crosslinked organopolysiloxane is preferably swelled by incorporating the liquid oil in an amount of its own weight or more. For the liquid oil agent, it is possible to use liquid silicone oil, hydrocarbon oil, ester oil, natural animal or vegetable oil, semisynthetic oil, or fluorine-based oil, which belongs to an optional component (1) oil agent. Examples thereof include Cyclopentasiloxane (INCI), Dimethicone (INCI), Mineral Oil (INCI), Isododecane (INCI), Isohexadecane (INCI), Triethylhexanoin (INCI), isotridecyl isononanoate (: Labeling Name (INCI: Isotridecyl Isononanoate)), Squalane (INCI), etc.

Different from a crosslinked silicone surfactant of the component (3) described above, the component (5) is a compound having no polyether or polyglycerol structure in the molecular structure, and specific examples thereof include (Dimethicone/Vinyl Dimethicone) Crosspolymer (INCI), (Dimethicone/Phenyl Vinyl Dimethicone) Crosspolymer (INCI), (Vinyl Dimethicone/Lauryl Dimethicone) Crosspolymer (INCI), (Lauryl Polydimethylsiloxyethyl Dimethicone/Bis-Vinyldimethicone) Crosspolymer (INCI), etc.

Example of commercial products of the composition including a crosslinked organopolysiloxane and an oil agent in a liquid state at room temperature include those manufactured by Shin-Etsu Chemical Co., Ltd.: KSG-15, KSG-1510, KSG-16, KSG-1610, KSG-19, KSG-016F, KSG-18A, KSG-41A, KSG-42A, KSG-43, KSG-44, KSG-042Z, KSG-045Z, KSG-048Z, etc.

### (6) Film-forming agent

The film-forming agent is contained mainly to maintain further persistence of the effect of the cosmetic. The film-forming agent is not particularly limited but is preferably a silicone-based composition from the viewpoint of imparting water repellency. Specifically, it is possible to use trimethylsiloxysilicate, acrylic-silicone film-forming agent, silicone-modified norbornene, silicone-modified pullulan, silicone-modified polyvinyl alcohol, or the like.

Examples of the film-forming agent of the silicone-based composition include trimethylsiloxysilicate (: Labeling Name (INCI: Trimethylsiloxysilicate)), (Acrylates/Dimethicone) Copolymer (INCI), (Norbornene/Tris(Trimethylsiloxy) Silylnorbornene) Copolymer (INCI), tri(trimethylsiloxy)silylpropyl carbamoyl pullulan (: Labeling Name (INCI: Trimethylsiloxysilylcarbamoyl Pullulan)), etc.

The film-forming agent may be contained in the cosmetic after being dissolved in an oil agent in a liquid state at room temperature. For the liquid oil agent, it is possible to use silicone oil, hydrocarbon oil, ester oil, natural animal or vegetable oil, semisynthetic oil, or fluorine-based oil, which belong to the optional component (1) oil agent.

Examples of the commercial product of silicon film-forming agents include those manufactured by Shin-Etsu Chemical Co., Ltd.: KF-7312J, KP-545, KP-549, KP-543, NBN-30-ID, TSPL-30-ID, TSPL-30-D5, etc.

### (7) Ultraviolet absorbing-scattering agent

Examples of the ultraviolet absorbing-scattering agent include particles that absorb and/or scatter ultraviolet light, such as titanium oxide fine particles, iron-containing titanium oxide fine particles, zinc oxide fine particles, cerium oxide fine particles, and composites thereof. It is also possible to use dispersion in which these particles that absorb and/or scatter ultraviolet light are dispersed in an oil agent in advance.

For the oil agent, it is possible to use liquid silicone oil, hydrocarbon oil, ester oil, natural animal or vegetable oil, semisynthetic oil, or fluorine-based oil, which belong to the optional component (1) oil agent.

Specific examples of the dispersion in which the particles that absorb and/or scatter ultraviolet light are dispersed in an oil agent in advance include SPD series (product name) manufactured by Shin-Etsu Chemical Co., Ltd., particularly SPD-T5, Z5, T6, Z6, T7, etc.

### (8) Other additives

Examples of the other additive include an oil-soluble gellant, an antiseptic/bactericide, an antiperspirant, a fragrance, a salt, an antioxidant, a pH adjuster, a chelator, a refrigerant, an anti-inflammatory agent, a skin-beautifying ingredient (a skin-whitening agent, a cell activator, a rough skin-improving agent, a blood circulation promoter, a skin astringent, an antiseborrheic agent, etc.), vitamins, amino acids, nucleic acids, hormone, an inclusion compound, etc.

### · Oil-soluble gellant

Examples of the oil-soluble gellant include: metal soap such as aluminum stearate, magnesium stearate, and zinc myristate; amino acid derivatives such as lauroyl glutamic acid (: Labeling Name (INCI: Lauroyl Glutamic Acid)) and α,γ-di-n-butylamine; dextrin fatty acid esters such as dextrin palmitate (: Labeling Name (INCI: Dextrin Palmitate)), dextrin isostearate (: Labeling Name (INCI: Dextrin Isostearate)), dextrin myristate (: Labeling Name (INCI: Dextrin Myristate)), stearoyl inulin (: Labeling Name (INCI: Stearoyl Inulin)), and dextrin (palmitate/ethylhexanoate) (: Labeling Name (INCI: Dextrin Palmitate/Ethylhexanoate)); sucrose fatty acid esters such as sucrose palmitic acid ester and sucrose stearic acid ester; fructooligosaccharide fatty acid ester such as fructooligosaccharide stearate ester and fructooligosaccharide 2-ethylhexanoate; benzylidene derivative of sorbitol such as monobenzylidene sorbitol and dibenzylidene sorbitol; Disteardimonium Hectorite (INCI), Stearalkonium Hectorite (INCI), organically modified clay mineral of hectorite; and Stearalkonium Bentonite (INCI); etc.

### · Antiseptic/bactericide

Examples of the antiseptic/bactericide include p-oxybenzoic acid alkyl ester, benzoic acid, sodium benzoate, sorbic acid, potassium sorbate, phenoxyethanol, imidazolidinylurea, salicylic acid, isopropyl methylphenol, carbolic acid, para chloro meta cresol, hexachlorophene, benzalkonium chloride, chlorhexidine chloride, trichlorocarbanilide, iodopropynyl butylcarbamate, polylysine, photosensitizer, silver, and plant extract, etc.

### · Antiperspirant

Examples of the antiperspirant include aluminum hydroxyhalide such as chlorohydroxy AL; aluminum halide such as AL chloride; aluminum allantoinate, tannic acid; persimmon tannin; (AL/K) sulfate; zinc oxide; zinc para-phenolsulfonate; burnt alum; (Al/zirconium) tetrachlorohydrate; (Al/zirconium) trichlorohydrex glycine; etc. In particular, as a component exhibiting a high effect, the preferable antiperspirants are aluminum hydroxyhalide, aluminum halide, and a complex or mixture thereof with zirconyl oxyhalide and zirconyl hydroxyhalide, which is such as (Al/zirconium) tetrachlorohydrate, and (Al/zirconium) trichlorohydrex glycine.

### Fragrance

The fragrance includes natural fragrance and synthetic fragrance. Examples of natural fragrances include vegetable fragrance separated from a flower, a leaf, wood, a peel, etc.; and animal fragrance such as musk and civet. Examples of the synthetic fragrance include hydrocarbons such as monoterpene; alcohols such as aliphatic alcohol and aromatic alcohol; aldehydes such as terpene aldehyde and aromatic aldehyde; ketones such as alicyclic ketone; esters such as terpene-based ester; lactones; phenols; oxides; nitrogen-containing compounds; acetals; etc.

### · Salt

Examples of the salts include an inorganic salt, an organic acid salt, an amine salt, and an amino acid salt. Examples of the inorganic salt include a sodium salt, a potassium salt, a magnesium salt, a calcium salt, an aluminum salt, a zirconium salt, a zinc salt, etc. of inorganic acids such as hydrochloric acid, sulfuric acid, carbonic acid, and nitric acid. Examples of the organic acid salt include a salt of organic acid such as acetic acid, dehydroacetic acid, citric acid, malic acid, succinic acid, ascorbic acid, and stearic acid. Examples of the amine salt and the amino acid salt include a salt of amine such as triethanolamine; and a salt of amino acid such as glutamic acid. Besides, it is also possible to use a salt of hyaluronic acid, chondroitin sulfate, etc.; aluminum zirconium glycine complex, etc.; an acid-alkali neutralization salt used in preparation of the cosmetics; etc.

### · Antioxidant

Examples of the antioxidant include, but are not particularly limited to, carotenoid, ascorbic acid and salt thereof, ascorbyl stearate, tocophenol acetate, tocophenol, p-t-butylphenol, butylhydroxyanisol, dibutylhydroxytoluene, phytic acid, ferulic acid, thiotaurine, hypotaurine, sulfite, erythorbic acid and salt thereof, chlorogenic acid, epicatechin, epigallocatechin, epigallocatechin gallate, apigenin, campherol, myricetin, quercetin, etc. One kind of the antioxidants may be used alone, or two or more kinds thereof may be used in combination.

### · pH adjuster

Examples of the pH adjuster include lactic acid, citric acid, glycolic acid, succinic acid, tartaric acid, dl-malic acid, potassium carbonate, sodium bicarbonate, ammonium bicarbonate, etc.

### Chelator

Examples of the chelator include alanine, sodium edetate, sodium polyphosphate, sodium metaphosphate, phosphoric acid, etc.

### Cooling agent

Examples of the cooling agent include L-menthol, camphor, menthyl lactate, etc.

### · Anti-inflammatory agent

Examples of the anti-inflammatory agent include allantoin, glycyrrhizinic acid and salt thereof, glycyrrhetinic acid and stearyl glycyrrhetinate, tranexamic acid, azulene, etc.

### · Skin-beautifying ingredient

Examples of the skin-beautifying ingredient include: skin-whitening agents such as placenta extract, arbutin, glutathione, and strawberry geranium extract; cell activators such as royal jelly, photosensitizer, cholesterol derivative, and calf blood extract; rough skin-improving agents; blood circulation promoters such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-orizanol; skin astringents such as zinc oxide and tannic acid; antiseborrheic agents such as sulfur and thianthrol; etc.

### · Vitamin

Examples of the vitamins include: vitamin A such as vitamin A oil, retinol, retinol acetate, and retinol palmitate; vitamin B2 such as riboflavin, riboflavin butyrate, and flavin adenine nucleotide; vitamin B6 such as pyridoxine hydrochloride, pyridoxine dioctanoate, and pyridoxine tripalmitate; vitamin B such as vitamin B12 and derivative thereof, and vitamin B15 and derivative thereof; vitamin C such as L-ascorbic acid, L-ascorbic acid dipalmitate, sodium L-ascorbic acid-2-sulfate, and L-ascorbic acid diester dipotassium phosphate; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and amide nicotinate; vitamin H; vitamin P; pantothenic acids such as calcium pantothenate, D-pantothenyl alcohol, pantothenyl ethyl ether, and acetyl pantothenyl ethyl ether; biotin; etc.

### Amino acid

Examples of the amino acids include glycine, valine, leucine, isoleucine, serine, threonine, phenylalanine, arginine, lysine, aspartic acid, glutamic acid, cystine, cysteine, methionine, tryptophan, etc.

### Nucleic acid

Examples of the nucleic acids include deoxyribonucleic acid, etc.

### Hormone

Examples of the hormone include estradiol, ethenyl estradiol, etc.

### · Inclusion compound

Examples of the inclusion compounds include cyclodextrin, etc.

### EXAMPLE

Hereinafter, the present invention will be specifically described with reference to Examples and Comparative Examples. However, the present invention is not limited thereto. Note that, in the examples, the kinematic viscosity is the value measured using a capillary viscometer at 25°C, and "%" representing concentration and content rate indicates "mass%."

### [Production Example 1]

### (Production of aqueous dispersion of silicone rubber particle)

344 g of vinyl group-containing dimethylpolysiloxane (A1) represented by the following formula (11) and having a kinematic viscosity of 55 mm²/s, 56 g (blending amount of 1.15 hydrosilyl groups relative to 1 vinyl group) of methylhydrogenpolysiloxane (B1) represented by the following formula (12) and having a kinematic viscosity of 30 mm²/s, and 0.1 g of dl-α-tocopherol (antioxidant) were charged into a 1.0-liter glass beaker, and were mixed and dissolved by using a homomixer. When the mixture was added with 30 g of polyoxyethylene lauryl ether (product name: EMULGEN 109P, manufactured by Kao Corporation) and 40 g of water and stirred using a homomixer, the viscosity increased to the point where it could be stirred no longer. The thickened product was kneaded for 15 minutes using a homodisper. Next, the thickened product was added with 528 g of water and mixed using a homomixer to obtain a uniform white emulsion. This emulsion was transferred to a 1-liter glass flask equipped with a stirring device using an anchor-shaped stirring blade, and the temperature was adjusted to 20 to 25°C. Then, with stirring, the emulsion was added with a dissolved mixture of 1 g of an isododecane solution (platinum content: 0.5%) of a platinum-vinyl group-containing disiloxane complex and 1 g of polyoxyethylene lauryl ether (product name: EMULGEN 109P, manufactured by Kao Corporation) and was stirred at the same temperature for 24 hours to obtain an aqueous dispersion of a silicone rubber particle.

The volume-average particle diameter of the silicone rubber particle was measured using "Laser Scattering Particle Size Distribution Analyzer LA-960" (manufacture by HORIBA, Ltd.) to be 300 nm.

Vinyl group-containing dimethylpolysiloxane (A1) represented by the following formula (11) and having a kinematic viscosity of 55 mm²/s, methylhydrogenpolysiloxane (B1) represented by the following formula (12) and having a kinematic viscosity of 30 mm²/s, and an isododecane solution (platinum content 0.5%) of platinum-vinyl group-containing disiloxane complex were mixed a blending ratio described above. The mixture was poured into an aluminum petri dish to have a thickness of 10 mm, left at 25°C for 6 hours, and further heated in a constant temperature bath at 50°C for 1 hour. The obtained cured product was a rubber elastic body without stickiness, and its hardness was 50 when measured using a durometer A hardness meter specified in JIS K6253.

### (A1): Vinyl group-containing dimethylpolysiloxane

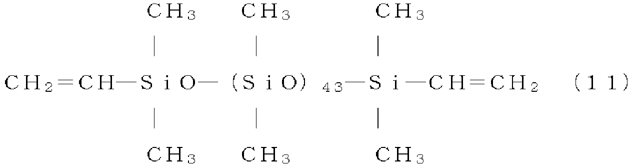

### (B1): Methylhydrogenpolysiloxane

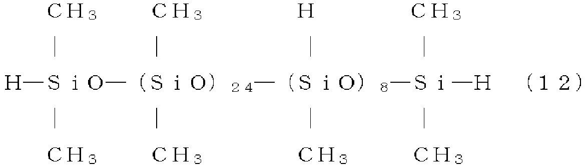

### [Production Example 2]

### (Production of aqueous dispersion of silicone rubber particles)

393g of vinyl group-containing dimethylpolysiloxane (A2) represented by the following formula (13) and having a kinematic viscosity of 5,060 mm²/s, 7 g (blending amount of 1.19 hydrosilyl groups relatively to 1 vinyl group) of methylhydrogenpolysiloxane represented by the following formula (12) and having a kinematic viscosity of 30 mm²/s, and 0.1 g of dl-α-tocopherol (antioxidant) were charged into a 1.0-liter glass beaker, and were mixed and dissolved by using a homomixer. When the mixture was added with 42 g of polyoxyethylene lauryl ether (product name: EMULGEN 109P, manufactured by Kao Corporation) and 35 g of water and stirred using a homomixer, the viscosity increased to the point where it could be stirred no longer. The thickened product was kneaded for 15 minutes using a homodisper. Next, the thickened product was added with 521 g of water and mixed using a homomixer to obtain a uniform white emulsion. This emulsion was transferred to a 1-liter glass flask equipped with a stirring device using an anchor-shaped stirring blade, and the temperature was adjusted to 20 to 25°C. Then, with stirring, the emulsion was added with a dissolved mixture of 0.6 g of an isododecane solution (platinum content: 0.5%) of a platinum-vinyl group-containing disiloxane complex and 1 g of polyoxyethylene lauryl ether (product name: EMULGEN 109P, manufactured by Kao Corporation) and was stirred at the same temperature for 24 hours to obtain an aqueous dispersion of silicone rubber particles.

The volume-average particle diameter of the silicone rubber particles was measured using "Laser Scattering Particle Size Distribution Analyzer LA-960" (manufacture by HORIBA, Ltd.) to be 300 nm.

Vinyl group-containing dimethylpolysiloxane represented by the following formula (13) and having a kinematic viscosity of 5,060 mm²/s, methylhydrogenpolysiloxane represented by the above formula (12) and having a kinematic viscosity of 30 mm²/s, and an isododecane solution (platinum content 0.5%) of platinum-vinyl group-containing disiloxane complex were mixed a blending ratio described above. The mixture was poured into an aluminum petri dish to have a thickness of 10 mm, left at 25°C for 6 hours, and further heated in a constant temperature bath at 50°C for 1 hour. The obtained cured product was a rubber elastic body without stickiness, and its hardness was 20 when measured using a durometer A hardness meter specified in JIS K6253.

### (A2): Vinyl group-containing dimethylpolysiloxane

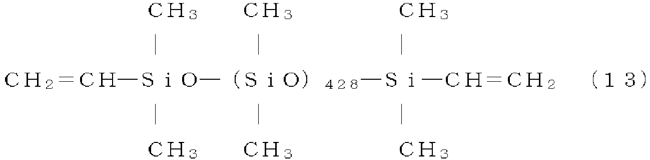

### [Example 1]

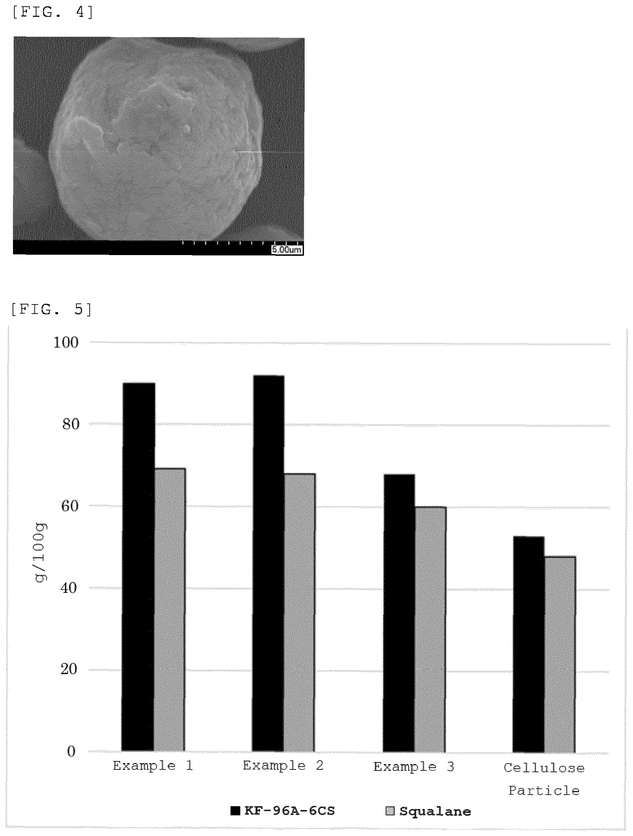

Into a 2-liter glass flask equipped with a stirring device using an anchor-shaped stirring blade, 150 g of a cellulose particle (product name: CELLULOBEADS D5, manufactured by Daito Kasei Kogyo Co., Ltd., shape: spherical, volume-average particle diameter: 12 µm, electron micrograph: Fig. 4), 20 g of the silicone rubber particle aqueous dispersion obtained in Production Example 1 (an amount in which the silicone rubber particle is 5.3 parts by mass relative to 100 parts by mass of the cellulose particle), and 5.5 g of a 30% lauryl trimethylammonium chloride aqueous solution (product name: Cation BB, manufactured by NOF CORPORATION) (an amount in which lauryltrimethylammonium chloride is 0.2 parts by mass relative to 100 parts by mass of water), 0.62 g a 40% dimethyldiallylammonium chloride polymer aqueous solution (product name: ME Polymer H40W, manufactured by Toho Chemical Industry Co., Ltd.) (amount in which dimethyldiallylammonium chloride polymer is 0.03 parts by mass relative to 100 parts by mass of water), 1.6 g of 2.8% ammonia water, and 800 g of water were charged. At this time, the pH of the mixture was 10.8. After adjusting the temperature to 5 to 10°C, 22 g of tetramethoxysilane (an amount in which the amount of silica is 8.69 g after the hydrolysis/condensation reaction, and silica is 109 parts by mass relative to 100 parts by mass of the silicone rubber particle after the hydrolysis/condensation reaction) was added dropwise to the mixture over 20 minutes with maintaining the temperature of the mixture at 5 to 10°C, and the mixture was stirred for another hour. Next, the mixture was heated to 75 to 80°C and stirred for 1 hour with maintaining that temperature to complete the hydrolysis/condensation reaction of tetramethoxysilane. The obtained suspension was dehydrated using a pressure filter. The dehydrated product was transferred to a 2-liter glass flask equipped with a stirring device using an anchor-type stirring blade, was added with 1,000 g of water, stirred for 30 minutes, and then dehydrated using a pressure filter. This operation was repeated twice. The obtained dehydrated product was dried in a hot-air fluidized bed dryer at a temperature of 105°C, and the dried product was crushed in a jet mill to obtain a particle.

The volume-average particle diameter of the obtained silicone particle was measured using an electrical resistance method particle size distribution analyzer (Multisizer 3, manufactured by BECKMAN COULTER) to be 12 µm.

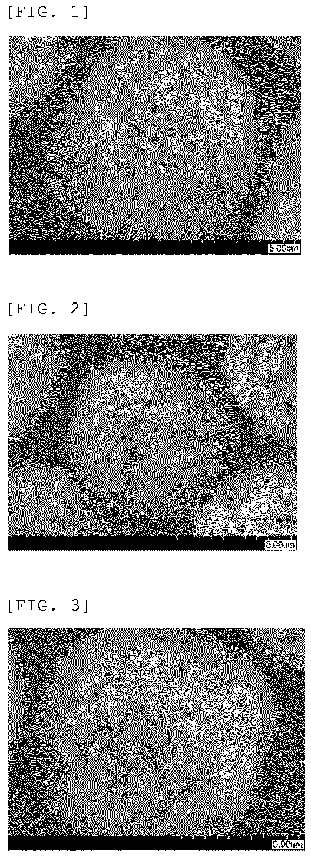

When the obtained particle was observed using an electron microscope, the surface of a spherical particle is covered with adhered spherical particles having a size of around 300 nm without any gaps, and it was confirmed to be a composite particle with silicone rubber particles adhered to the surface of the cellulose particle. An electron micrograph is shown in FIG. 1.

5 g of the obtained particle was added to a 100 mL-beaker containing 80 g of a 1% aqueous solution of polyoxyethylene lauryl ether (product name: EMULGEN 109P, manufactured by Kao Corporation), and was stirred with a glass rod to be dispersed in the aqueous solution. After standing still for 24 hours, the floating of the particles was observed, and the particles were all found to be settled. Silicone rubber particles float because their specific gravity is lower than water, but from this result it was determined that the silicone rubber particle did not fall off the surface of the cellulose particle and it was indicated that silica acts as a binder and causes the silicone rubber particle to fix to the surface of the cellulose particle.

### [Example 2]

Into a 2-liter glass flask equipped with a stirring device using an anchor-shaped stirring blade, 150 g of a cellulose particle (product name: CELLULOBEADS D5, manufactured by Daito Kasei Kogyo Co., Ltd., shape: spherical, volume-average particle diameter: 12 µm), 20 g of the silicone rubber particle aqueous dispersion obtained in Production Example 2 (an amount in which the silicone rubber particle is 5.3 parts by mass relative to 100 parts by mass of the cellulose particle), and 5.5 g of a 30% lauryl trimethylammonium chloride aqueous solution (product name: Cation BB, manufactured by NOF CORPORATION) (an amount in which lauryltrimethylammonium chloride is 0.2 parts by mass relative to 100 parts by mass of water), 0.62 g a 40% dimethyldiallylammonium chloride polymer aqueous solution (product name: ME Polymer H40W, manufactured by Toho Chemical Industry Co., Ltd.) (amount in which dimethyldiallylammonium chloride polymer is 0.03 parts by mass relative to 100 parts by mass of water), 1.6 g of 2.8% ammonia water, and 811 g of water were charged. At this time, the pH of the mixture was 10.8. After adjusting the temperature to 5 to 10°C, 11 g of tetramethoxysilane (an amount in which the amount of silica is 4.34 g after the hydrolysis/condensation reaction, and silica is 54 parts by mass relative to 100 parts by mass of the silicone rubber particle after the hydrolysis/condensation reaction) was added dropwise to the mixture over 10 minutes with maintaining the temperature of the mixture at 5 to 10°C, and the mixture was stirred for another hour. Next, the mixture was heated to 75 to 80°C and stirred for 1 hour with maintaining that temperature to complete the hydrolysis/condensation reaction of tetramethoxysilane. The obtained suspension was dehydrated using a pressure filter. The dehydrated product was transferred to a 2-liter glass flask equipped with a stirring device using an anchor-type stirring blade, was added with 1,000 g of water, stirred for 30 minutes, and then dehydrated using a pressure filter. This operation was repeated twice. The obtained dehydrated product was dried in a hot-air fluidized bed dryer at a temperature of 105°C, and the dried product was crushed in a jet mill to obtain a particle.

The volume-average particle diameter of the obtained silicone particle was measured using an electrical resistance method particle size distribution analyzer (Multisizer 3, manufactured by BECKMAN COULTER) to be 12 µm.

When the obtained particle was observed using an electron microscope, 80 to 90% of the surface of the spherical particle is covered with adhered spherical particles having a size of around 300 nm and their aggregates, and it was confirmed to be a composite particle with silicone rubber particles adhered to the surface of the cellulose particle. An electron micrograph is shown in FIG. 2.

5 g of the obtained particle was added to a 100 mL-beaker containing 80 g of a 1% aqueous solution of polyoxyethylene lauryl ether (product name: EMULGEN 109P, manufactured by Kao Corporation), and was stirred with a glass rod to be dispersed in the aqueous solution. After standing still for 24 hours, the floating of the particles was observed, and the particles were all found to be settled. Silicone rubber particles float because their specific gravity is lower than water, but from this result it was determined that the silicone rubber particles did not fall off the surface of the cellulose particle and it was indicated that silica acts as a binder and causes the silicone rubber particles to fix to the surface of the cellulose particle.

### [Example 3]

Into a 2-liter glass flask equipped with a stirring device using an anchor-shaped stirring blade, 150 g of a cellulose particle (product name: CELLULOBEADS D5, manufactured by Daito Kasei Kogyo Co., Ltd., shape: spherical, volume-average particle diameter: 12 µm), 3.5 g of the silicone rubber particle aqueous dispersion obtained in Production Example 2 (an amount in which the silicone rubber particle is 0.9 parts by mass relative to 100 parts by mass of the cellulose particle), and 5.6 g of a 30% lauryl trimethylammonium chloride aqueous solution (product name: Cation BB, manufactured by NOF CORPORATION) (an amount in which lauryltrimethylammonium chloride is 0.2 parts by mass relative to 100 parts by mass of water), 0.63 g a 40% dimethyldiallylammonium chloride polymer aqueous solution (product name: ME Polymer H40W, manufactured by Toho Chemical Industry Co., Ltd.) (amount in which dimethyldiallylammonium chloride polymer is 0.03 parts by mass relative to 100 parts by mass of water), 1.6 g of 2.8% ammonia water, and 828 g of water were charged. At this time, the pH of the mixture was 10.8. After adjusting the temperature to 5 to 10°C, 11 g of tetramethoxysilane (an amount in which the amount of silica is 4.34 g after the hydrolysis/condensation reaction, and silica is 310 parts by mass relative to 100 parts by mass of the silicone rubber particle after the hydrolysis/condensation reaction) was added dropwise to the mixture over 10 minutes with maintaining the temperature of the mixture at 5 to 10°C, and the mixture was stirred for another hour. Next, the mixture was heated to 75 to 80°C and stirred for 1 hour with maintaining that temperature to complete the hydrolysis/condensation reaction of tetramethoxysilane. The obtained suspension was dehydrated using a pressure filter. The dehydrated product was transferred to a 2-liter glass flask equipped with a stirring device using an anchor-type stirring blade, was added with 1,000 g of water, stirred for 30 minutes, and then dehydrated using a pressure filter. This operation was repeated twice. The obtained dehydrated product was dried in a hot-air fluidized bed dryer at a temperature of 105°C, and the dried product was crushed in a jet mill to obtain a particle.

The volume-average particle diameter of the obtained silicone particle was measured using an electrical resistance method particle size distribution analyzer (Multisizer 3, manufactured by BECKMAN COULTER) to be 12 µm.

When the obtained particle was observed using an electron microscope, the surface of the spherical particle is sparsely adhered with spherical particles having a size of around 300 nm, and it was confirmed to be a composite particle with silicone rubber particles adhered to the surface of the cellulose particle. On the surface to which no silicone rubber particles were adhered, spherical particles having a size of around 100 nm or less was adhered and determined to be silica. An electron micrograph is shown in FIG. 3.

5 g of the obtained particle was added to a 100 mL-beaker containing 80 g of a 1% aqueous solution of polyoxyethylene lauryl ether (product name: EMULGEN 109P, manufactured by Kao Corporation), and was stirred with a glass rod to be dispersed in the aqueous solution. After standing still for 24 hours, the floating of the particles was observed, and the particles were all found to be settled. Silicone rubber particles float because their specific gravity is lower than water, but from this result it was determined that the silicone rubber particles did not fall off the surface of the cellulose particle and it was indicated that silica acts as a binder and causes the silicone rubber particles to fix to the surface of the cellulose particle.

### [Comparative Example 1]

Into a 2-liter glass flask equipped with a stirring device using an anchor-shaped stirring blade, 150 g of a cellulose particle (product name: CELLULOBEADS D5, manufactured by Daito Kasei Kogyo Co., Ltd., shape: spherical, volume-average particle diameter: 12 µm), 20 g of the silicone rubber particle aqueous dispersion obtained in Production Example 1 (an amount in which the silicone rubber particle is 5.3 parts by mass relative to 100 parts by mass of the cellulose particle), 1.6 g of 2.8% ammonia water, and 806 g of water were charged. At this time, the pH of the mixture was 10.8. After adjusting the temperature to 5 to 10°C, 22 g of tetramethoxysilane (an amount in which the amount of silica is 8.69 g after the hydrolysis/condensation reaction, and silica is 109 parts by mass relative to 100 parts by mass of the silicone rubber particle after the hydrolysis/condensation reaction) was added dropwise to the mixture over 20 minutes with maintaining the temperature of the mixture at 5 to 10°C, and the mixture was stirred for another hour. Next, the mixture was heated to 75 to 80°C and stirred for 1 hour with maintaining that temperature to complete the hydrolysis/condensation reaction of tetramethoxysilane. The obtained suspension was dehydrated using a pressure filter. The dehydrated product was transferred to a 2-liter glass flask equipped with a stirring device using an anchor-type stirring blade, was added with 1,000 g of water, stirred for 30 minutes, and then dehydrated using a pressure filter. This operation was repeated twice. The obtained dehydrated product was dried in a hot-air fluidized bed dryer at a temperature of 105°C, and the dried product was crushed in a jet mill to obtain a particle.

When the obtained particle was observed using an electron microscope, the surface of the cellulose particle is not adhered with particles.

In the process of adding the tetraalkoxysilane to the liquid mixture of the cellulose particle, the silicone rubber particle, the aqueous ammonia, and water and causing the hydrolysis/condensation reaction, when the cationic substance (the cationic surfactant and/or the cationic water-soluble polymer) is not blended, the silicone rubber particles do not adhere to the surface of the cellulose particle.

### [Measurement of Oil Absorption Amount of Composite Particle]

Regarding the composite particles produced in the above Examples and the cellulose particle (product name: CELLULOBEADS D5, manufactured by Daito Kasei Kogyo Co., Ltd., shape: spherical, volume-average particle diameter: 12 µm), the oil absorption amount was measured using an oil agent used for cosmetics, with reference to "JIS K 5101-13-1: Part 13: Oil absorption - Section 1: Refined linseed oil method." The results are shown in Table 1 and FIG. 5 below.

**[Table 1]**

| Item | | | Example 1 | Example 2 | Example 3 | Comparative Example 1* | Cellulose particle |
|---|---|---|---|---|---|---|---|
| Spherical organic resin particle | | Organic resin | Cellulose | Cellulose | Cellulose | Cellulose | Cellulose |
| | | Average particle diameter (µm) | 12 | 12 | 12 | 12 | 12 |
| Rubber particle | | Rubber | Silicone rubber | Silicone rubber | Silicone rubber | Silicone rubber | - |
| | | Average particle diameter (nm) | 300 | 300 | 300 | 300 | - |
| | | Hardness Durometer A | 50 | 20 | 20 | 50 | - |
| Weight ratio Organic resin particle / Rubber particle | | | 100/5.3 | 100/5.3 | 100/0.9 | 100/5.3 | 100/0 |
| Weight ratio Rubber particle / Silica | | | 100/109 | 100/54 | 100/310 | 100/109 | - |
| Oil absorption g/100g | KF-96A-6cs (Note 1) | | 90 | 92 | 68 | - | 53 |
| | Squalane | | 69 | 68 | 60 | - | 48 |

| | | | | | | | |
|---|---|---|---|---|---|---|---|
| (Note 1) Manufactured by Shin-Etsu Chemical Co., Ltd.: dimethicone (INCI) with a kinematic viscosity of 6 mm²/s at 25°C *In Comparative Example 1, the rubber particle and the silica did not adhere to the organic resin particle. | | | | | | | |

From the results shown in Table 1 and FIG. 5 above, it was found that Examples 1 to 3 had higher oil-absorbing properties than the cellulose particle for respective oil agents. Among them, in Example 3, the amount of the silicone rubber particle was 1.0 parts by mass or less per 100 parts by mass of the organic resin particle, but it was found that the oil-absorbing property for respective oil agents was high.

### [Examples 4 to 6, Comparative Example 2: Usability Evaluation of Composite Particle]

Skin care cosmetics having the formulation shown in Table 3 below were prepared by the following method, and the following characterization evaluation was performed.

### (1) Usability evaluation

Regarding the skin care cosmetics containing the composite particle prepared in the above examples and the cellulose particle (product name: CELLULOBEADS D5, manufactured by Daito Kasei Kogyo Co., Ltd., shape: spherical, volume-average particle diameter: 12 µm), the usability in applying (spreadability, affinity with skin) and the usability after applying (no stickiness, blurring, smoothness) were evaluated. The degree of usability was evaluated by 10 expert panelists. Evaluation was made according to the evaluation criteria shown in Table 2, and the results were judged according to the following criteria based on the average value of 10 expert panelists. The results are shown in Table 3 below.

**[Table 2]**

| Item | Degree of usability |
|---|---|
| 5 points | Good |
| 4 points | Somewhat good |
| 3 points | Average |
| 2 points | Somewhat bad |
| 1 points | Bad |

### (2) Judgment criteria of usability

Excellent: The obtained average is 4.0 points or more
Good: The obtained average is 3.0 point or more and less than 4.0
Fair: The obtained average is 2.0 point or more and less than 3.0
Poor: The obtained average is less than 2.0

**[Table 3]**

| Composition (%) | | | Example 4 | Example 5 | Example 6 | Comparative Example 2 |
|---|---|---|---|---|---|---|
| KSG-210 (Note 1) | | | 3.0 | 3.0 | 3.0 | 3.0 |
| KSG-19 (Note 2) | | | 1.0 | 1.0 | 1.0 | 1.0 |
| KF-6017 (Note 3) | | | 0.2 | 0.2 | 0.2 | 0.2 |
| KF-96A-6cs (Note 4) | | | 12.0 | 12.0 | 12.0 | 12.0 |
| Composite particle obtained in Example 1 | | | 5.0 | | | |
| Composite particle obtained in Example 2 | | | | 5.0 | | |
| Composite particle obtained in Example 3 | | | | | 5.0 | |
| Cellulose particle | | | | | | 5.0 |
| Glycerin | | | 8.0 | 8.0 | 8.0 | 8.0 |
| BG | | | 4.0 | 4.0 | 4.0 | 4.0 |
| Phenoxyethanol | | | 0.3 | 0.3 | 0.3 | 0.3 |
| Sodium citrate | | | 0.2 | 0.2 | 0.2 | 0.2 |
| Sodium chloride | | | 0.5 | 0.5 | 0.5 | 0.5 |
| Water | | | 65.8 | 65.8 | 65.8 | 65.8 |
| Sum | | | 100.0 | 100.0 | 100.0 | 100.0 |
| Evaluation | Usability in applying | Spreadability | Good | Good | Good | Good |
| | | Affinity with skin | Excellent | Excellent | Good | Fair |
| | Usability after applying | No stickiness | Excellent | Excellent | Good | Fair |
| | | Blurring | Excellent | Excellent | Good | Fair |
| | | Smoothness | Excellent | Excellent | Good | Fair |

| | | | | | | |
|---|---|---|---|---|---|---|
| (Note 1) Manufactured by Shin-Etsu Chemical Co., Ltd.: mixture of (dimethicone 70-80% + (dimethicone/(PEG-10/15)) crosspolymer 20-30%) (Note 2) Manufactured by Shin-Etsu Chemical Co., Ltd.: mixture of (dimethicone 80-90% + (dimethicone/vinyl dimethicone) crosspolymer 10-20%) (Note 3) Manufactured by Shin-Etsu Chemical Co., Ltd.: PEG-10 dimethicone (Note 4) Manufactured by Shin-Etsu Chemical Co., Ltd.: dimethicone having a kinematic viscosity of 6 mm²/s at 25°C | | | | | | |

The results in Table 3 above show that Examples 1 to 3 have higher oil-absorbing properties than the cellulose particle. Further, because they are composite particles with silicone rubber particles adhered to the surface, Examples 4 to 6 being cosmetics containing Examples 1 to 3 had good affinity with the skin in applying, and in usability after applying they are evaluated to be smooth and higher in blurring than those of Comparative Example 2.

### Example 7: Water-in-oil cream

| Component | (%) |
|---|---|
| 1. KSG-310 (note 1) | 3.0 |
| 2. KSG-44 (note 2) | 1.0 |
| 3. KF-6048 (note 3) | 0.2 |
| 4. Scualane | 10.8 |
| 5. Composite particle obtained in Example 3 | 1.0 |
| 6. BG | 8.0 |
| 7. Ethanol | 5.0 |
| 8. Magnesium sulfate | 0.2 |
| 9. Sodium chloride | 0.5 |
| 10. Water | Residual amount |
| Total | 100.0 |

| | |
|---|---|
| (Note 1) Manufactured by Shin-Etsu Chemical Co., Ltd.: mixture of (mineral oil 65-75% + (PEG-15/lauryl dimethicone) crosspolymer 25-35%) (Note 2) Manufactured by Shin-Etsu Chemical Co., Ltd.: Mixture of (squalane 65-75% + (vinyl dimethicone/lauryl dimethicone) crosspolymer 25-35%) (Note 3) Manufactured by Shin-Etsu Chemical Co., Ltd.: cetyl PEG/PPG-10/1 Dimethicone | |

### (Manufacturing method)

A: Mix components 1 to 5.
B: Mix components 6 to 10.
C: Add the material obtained in step B above to the material obtained in step A above and mix uniformly.
D: After defoaming the product obtained in step C above, it is filled into a container to obtain water-in-oil cream.

The inventive water-in-oil cream obtained as described above had excellent smoothness in applying, was not sticky, spread easily, was excellent in feeling of adherence, settled well, and had a natural finish with a suppressed shine.

### Example 8: Liquid emulsion foundation

| Component | (%) |
|---|---|
| 1. KSG-710 (Note 1) | 4.0 |
| 2. KSG-15 (Note 2) | 2.0 |
| 3. KF-6105 (Note 3) | 3.0 |
| 4. KF-96A-6cs (Note 4) | 12.0 |
| 5. Disteardimonium hectorite | 1.2 |
| 6. Composite particle obtained in Example 3 | 3.0 |
| 7. KF-7312J (Note 5) | 5.0 |
| 8. Isotridecyl isononanoate | 2.0 |
| 9. KF-6106 (Note 6) | 0.5 |
| 10. KTP-09W (Note 7) | 8.5 |
| 11. KTP-09R (Note 7) | 0.4 |
| 12. KTP-09Y (Note 7) | 1.0 |
| 13. KTP-09B (Note 7) | 0.1 |
| 14. Fragrance | 0.1 |
| 15. Pentylene glycol | 5.0 |
| 16. Sodium citrate | 0.2 |
| 17. Sodium chloride | 0.5 |
| 18. Water | Residual amount |
| Total | 100.0 |

| | |
|---|---|
| (Note 1) Manufactured by Shin-Etsu Chemical Co., Ltd.: mixture of (dimethicone 70-80% + (dimethicone/polyglycerin-3) crosspolymer 20-30%) (Note 2) Manufactured by Shin-Etsu Chemical Co., Ltd.: mixture of (cyclopentasiloxane 90-96% + (dimethicone/vinyl dimethicone) crosspolymer 4-10%) (Note 3) Manufactured by Shin-Etsu Chemical Co., Ltd.: lauryl polyglyceryl-3 polydimethylsiloxyethyl dimethicone (Note 4) Manufactured by Shin-Etsu Chemical Co., Ltd.: dimethicone having a kinematic viscosity of 6 mm²/s at 25°C (Note 5) Manufactured by Shin-Etsu Chemical Co., Ltd.: dissolved product of 50% trimethylsiloxysilicic acid dissolved in cyclopentasiloxane (Note 6) Manufactured by Shin-Etsu Chemical Co., Ltd.: polyglyceryl-3 polydimethylsiloxyethyl dimethicone (Note 7) Manufactured by Shin-Etsu Chemical Co., Ltd.: colored inorganic pigment treated by triethoxysilylethyl polydimethylsiloxyethylhexyl dimethicone, W: white, R: red, Y: yellow, B: black | |

### (Manufacturing method)

A: Mix components 1 to 7.
B: Mix components 8 to 13, perform roll-treatment.
C: Add the material obtained in step B above to the material obtained in step A above and mix uniformly.
D: Mix components 14 to 18.
E: Add the material obtained in step D above to the material obtained in step C above and mix uniformly.
F: After defoaming the product obtained in step E above, it is filled into a container to obtain liquid emulsion foundation.

The inventive liquid emulsion foundation obtained as described above was excellent in smoothness in applying and moisture retaining property, was not sticky, spread easily, was excellent in feeling of adherence, settled well, and had a natural finish with a suppressed shine.

### Example 9: Powder foundation

| Component | (%) |
|---|---|
| 1. Neopentyl glycol diethylhexanoate | 4.0 |
| 2. Mineral oil | 2.0 |
| 3. KF-56A (Note 1) | 2.0 |
| 4. Dipentaerythrityl hexa (hydroxystearate/ stearate/rosinate) | 0.2 |
| 5. Zinc stearate | 1.0 |
| 6. Composite particle obtained in Example 3 | 5.0 |
| 7. Polymethylsilsesquioxane (Note 2) | 3.0 |
| 8. Boron nitride | 3.0 |
| 9. KF-99P treated mica (Note 3) | 20.0 |
| 10. KF-99P treated talc (Note 3) | Residual amount |
| 11. AES-3083 processed pigment grade titanium oxide (white) (Note 4) | 8.0 |
| 12. AES-3083 treated iron oxide (red) (Note 4) | 0.4 |
| 13. AES-3083 treated iron oxide (yellow) (Note 4) | 1.3 |
| 14. AES-3083 treated iron oxide (black) (Note 4) | 0.3 |
| Total | 100.0 |

| | |
|---|---|
| (Note 1) Manufactured by Shin-Etsu Chemical Co., Ltd.: diphenylsiloxyphenyl trimethicone (Note 2) Manufactured by Shin-Etsu Chemical Co., Ltd.: polymethylsilsesquioxane (Note 3) Manufactured by Shin-Etsu Chemical Co., Ltd.: methicone treatment (Note 4) Manufactured by Shin-Etsu Chemical Co., Ltd.: triethoxycaprylylsilane treatment | |

### (Manufacturing method)

A: Mix components 1 to 4 uniformly.
B: Mix components 5 to 14 uniformly.
C: Add the material obtained in step A above to the material obtained in step B above and mix.
D: Material obtained in step C above was passed through a sieve and then stamped into a metal plate using a mold to obtain powder foundation.

The inventive powder foundation obtained as described above was excellent in smoothness and adhesion property in applying, and had a finish with good cosmetic persistence.

### Example 10: Water-in-oil concealer

| Component | (%) |
|---|---|
| 1. KSG-210 (Note 1) | 3.0 |
| 2. KSG-15 (Note 2) | 5.0 |
| 3. KF-6028 (Note 3) | 2.0 |
| 4. KF-96L-2cs (Note 4) | Residual amount |
| 5. Composite particle obtained in Example 1 | 10.0 |
| 6. KP-545 (Note 5) | 3.0 |
| 7. Ethylhexyl palmitate | 2.0 |
| 8. KP-578 (Note 6) | 0.3 |
| 9. KTP-09W (Note 7) | 7.0 |
| 10. KTP-09R (Note 7) | 0.2 |
| 11. KTP-09Y (Note 7) | 0.8 |
| 12. KTP-09B (Note 7) | 0.2 |
| 13. BG | 5.0 |
| 14. Ethanol | 8.0 |
| 15. Sodium citrate | 0.2 |
| 16. Sodium chloride | 0.5 |
| 17. Water | 30.0 |
| Total | 100.0 |

| | |
|---|---|
| (Note 1) Manufactured by Shin-Etsu Chemical Co., Ltd.: mixture of (dimethicone 70-80% + (dimethicone/(PEG-10/15)) crosspolymer 20-30%) (Note 2) Manufactured by Shin-Etsu Chemical Co., Ltd.: mixture of (cyclopentasiloxane 90-96% + (dimethicone/vinyl dimethicone) crosspolymer 4-10%) (Note 3) Manufactured by Shin-Etsu Chemical Co., Ltd.: PEG-9 polydimethylsiloxyethyl dimethicone (Note 4) Manufactured by Shin-Etsu Chemical Co., Ltd.: dimethicone having a kinematic viscosity of 2 mm²/s at 25°C (Note 5) Manufactured by Shin-Etsu Chemical Co., Ltd.: dissolved product of 30% (acrylates/dimethicone) copolymer in cyclopentasiloxane (Note 6) Manufactured by Shin-Etsu Chemical Co., Ltd.: (acrylates/ethylhexyl acrylate/dimethicone methacrylate) copolymer (Note 7) Manufactured by Shin-Etsu Chemical Co., Ltd.: colored inorganic pigment treated by triethoxysilylethyl polydimethylsiloxyethylhexyl dimethicone, W: white, R: red, Y: yellow, B: black | |

### (Manufacturing method)

A: Mix components 1 to 6.
B: Mix components 7 to 12, perform roll-treatment.
C: Add the material obtained in step B above to the material obtained in step A above and mix uniformly.
D: Mix components 13 to 17.
E: Add the material obtained in step D above to the material obtained in step C above and mix uniformly.
F: After defoaming the product obtained in step E above, it is filled into a container to obtain water-in-oil concealer.

The inventive water-in-oil concealer obtained as described above did not have smoothness and stickiness in applying, was excellent in adhesiveness, suppressed a shine, and provided a finish with good cosmetic persistence.

### Example 11: Lipstick

| Component | (%) |
|---|---|
| 1. Polyethylene wax | 5.0 |
| 2. Ceresin | 2.0 |
| 3. Microcrystalline wax | 3.0 |
| 4. Candelilla wax | 1.0 |
| 5. Diisostearyl malate | 15.0 |
| 6. KP-561P (Note 1) | 2.0 |
| 7. Composite particle obtained in Example 3 | 12.0 |
| 8. Sorbitan sesquiisostearate | 1.0 |
| 9. Polyglyceryl triisostearate-2 | 15.0 |
| 10. Tri(caprylic acid/capric acid) glyceryl | Residual amount |
| 11. Triethylhexanoin | 3.0 |
| 12. KP-578 (Note 2) | 0.5 |
| 13. Red No. 202 | 0.5 |
| 14. Yellow No. 4 Aluminum Lake | 1.6 |
| 15. KP-574 treated pigment grade titanium oxide (white) (Note 3) | 4.0 |
| 16. KP-574 treated iron oxide (red) (Note 3) | 1.0 |
| 17. KP-574 treated iron oxide (yellow) (Note 3) | 0.3 |
| 18. KP-574 treated iron oxide (black) (Note 3) | 0.3 |
| 19. Titanium oxide coated mica pearl agent | 3.0 |
| Total | 100.0 |

| | |
|---|---|
| (Note 1) Manufactured by Shin-Etsu Chemical Co., Ltd.: (Acrylates/stearyl acrylate/dimethicone methacrylate) copolymer (Note 2) Manufactured by Shin-Etsu Chemical Co., Ltd.: (acrylates/ethylhexyl acrylate/dimethicone methacrylate) copolymer (Note 3) Manufactured by Shin-Etsu Chemical Co., Ltd.: (acrylates/tridecyl acrylate/triethoxysilylpropyl methacrylate/dimethicone methacrylate) copolymer treatment | |

### (Manufacturing method)

A: Components 1 to 10 were heated and dissolved.
B: Mix components 11 to 18 uniformly, and perform roll-treatment.
C: Add the material obtained in step B above and component 19 to the material obtained in step A above and mix uniformly.
D: After defoaming the product obtained in step C above, it is filled into a container to obtain lipstick.

The inventive lipstick obtained as described above had excellent smoothness in applying, was not sticky, spread easily, was excellent in adhesiveness, had good fitting, and provided a mat finish with a suppressed shine.

### Example 12: Oil-in-water base cream

| Component | (%) |
|---|---|
| 1. Water | Residual amount |
| 2. Glycerin | 3.0 |
| 3. Microcrystalline wax | 3.0 |
| 4. Xanthan gum | 0.2 |
| 5. Pentylene glycol | 2.0 |
| 6. BG | 5.0 |
| 7. Coconut fatty acid sucrose | 0.2 |
| 8. Sorbitan stearate | 3.0 |
| 9. PEG-60 glyceryl isostearate | 0.5 |
| 10. Behenyl alcohol | 0.5 |
| 11. Ethylhexyl palmitate | 3.0 |
| 12. Composite particle obtained in Example 2 | 3.0 |
| 13. Triethylhexanoin | 6.0 |
| 14. Polyhydroxystearic acid | 0.5 |
| 15. Titanium oxide fine particle treated with metal soap | 8.0 |
| 16. Pigment grade titanium oxide (white) treated with metal soap | 4.0 |
| 17. Iron oxide (red) treated with metal soap | 0.1 |
| 18. Iron oxide (yellow) treated with metal soap | 0.8 |
| 19. Iron oxide (black) treated with metal soap | 0.1 |
| 20. Polysorbate 60 | 0.3 |
| 21. (Hydroxyethyl acrylate/acryloyldimethyltaurin | |
| sodium) copolymer | 0.6 |
| Total | 100.0 |

### (Manufacturing method)

A: Mix components 1 to 6 uniformly.
B: Components 7 to 11 were heated and dissolved, added with component 12, and mixed uniformly.
C: Mix components 13 to 19, and perform roll-treatment.
D: Add the material obtained in step C above to the material obtained in step B above and mix uniformly.
E: Add the heated material obtained in step D above to the heated material obtained in step A above and mix uniformly.
F: After cooling the product obtained in step E above down to normal temperature, add components 20 to 21, and mix uniformly.
G: After defoaming the product obtained in step F above, it is filled into a container to obtain oil-in-water base cream.

The inventive oil-in-water base cream obtained as described above had excellent freshness in applying, was not sticky, spread easily, was excellent in adhesiveness, had good fitting, and provided a mat finish with a suppressed shine.

### Example 13: Aqueous gel

| Component | (%) |
|---|---|
| 1. Composite particle obtained in Example 3 | 5.0 |
| 2. KF-6100 (Note 1) | 0.5 |
| 3. Ethanol | 3.0 |
| 4. BG | 4.0 |
| 5. Glycerin | 2.0 |
| 6. (Acryloyldimethyltaurate ammonium/VP) copolymer | |
| | 0.2 |
| 7. Xanthan gum | 0.2 |
| 8. Arginine | 0.5 |
| 9. Phenoxyethanol | 0.3 |
| 10. Water | Residual amount |
| Total | 100.0 |

| | |
|---|---|
| (Note 1) Manufactured by Shin-Etsu Chemical Co., Ltd.: Polyglyceryl-3 disiloxane dimethicone | |

### (Manufacturing method)

A: Mix components 1 to 3 uniformly.
B: Mix components 4 to 10 uniformly.
C: Add the material obtained in step A above to the material obtained in step B above and mix uniformly.
D: After defoaming the product obtained in step C above, it is filled into a container to obtain aqueous gel.

The inventive aqueous gel obtained as described above had excellent freshness in applying, was not sticky, spread easily, was excellent in adhesiveness, had good fitting to the skin, and provided a mat finish with a suppressed shine.

### Example 14: Oily gel

| Component | (%) |
|---|---|
| 1. Composite particle obtained in Example 1 | 10.0 |
| 2. KSG-16 (Note 1) | 20.0 |
| 3. KSG-15 (Note 2) | 30.0 |
| 4. KF-56A (Note 3) | 5.0 |
| 5. KF-995 (Note 4) | Residual amount |
| Total | 100.0 |

| | |
|---|---|
| (Note 1) Manufactured by Shin-Etsu Chemical Co., Ltd.: mixture of (dimethicone 70-80% + (dimethicone/vinyl dimethicone) crosspolymer 20-30%) (Note 2) Manufactured by Shin-Etsu Chemical Co., Ltd.: mixture of (cyclopentasiloxane 90-96% + (dimethicone/vinyl dimethicone) crosspolymer 4-10%) (Note 3) Manufactured by Shin-Etsu Chemical Co., Ltd.: diphenylsiloxyphenyl trimethicone (Note 4) Manufactured by Shin-Etsu Chemical Co., Ltd.: cyclopentasiloxane | |

### (Manufacturing method)

A: Mix components 1 to 5 uniformly.
B: After defoaming the product obtained in step A above, it is filled into a container to obtain oily gel.

The inventive oily gel obtained as described above had excellent smoothness in applying, was not sticky, spread easily, was excellent in adhesiveness, had good fitting, and provided a mat finish with a suppressed shine.

### Example 15: Oil-based solid foundation

| Component | (%) |
|---|---|
| 1. Synthetic wax | 4.0 |
| 2. Carnauba wax | 2.0 |
| 3. Shea butter | 0.5 |
| 4. tri (Caprylic/capric) glyceride | 3.0 |
| 5. KF-56A (Note 1) | 5.0 |
| 6. Ethylhexyl methoxycinnamate | 7.0 |
| 7. Bis-ethylhexyloxyphenol methoxyphenyl triazinel | 0.5 |
| 8. KF-96L-2cs (Note 2) | 5.0 |
| 9. Isotridecyl isononanoate | Residual amount |
| 10. Composite particle obtained in Example 2 | 4.0 |
| 11. KMP-591 (Note 3) | 1.0 |
| 12. Cetyl ethylhexanoate | 3.0 |
| 13. KF-6115 (Note 4) | 1.0 |
| 14. KF-99P treated titanium oxide fine particle (Note 5) | 7.0 |
| 15. KTP-09W (Note 6) | 9.0 |
| 16. KTP-09R (Note 6) | 0.3 |
| 17. KTP-09Y (Note 6) | 1.0 |
| 18. KTP-09B (Note 6) | 0.2 |
| Total | 100.0 |

| | |
|---|---|
| (Note 1) Manufactured by Shin-Etsu Chemical Co., Ltd.: diphenylsiloxyphenyl trimethicone (Note 2) Manufactured by Shin-Etsu Chemical Co., Ltd.: dimethicone having a kinematic viscosity of 2 mm²/s at 25°C (Note 3) Manufactured by Shin-Etsu Chemical Co., Ltd.: polymethylsilsesquioxane (Note 4) Manufactured by Shin-Etsu Chemical Co., Ltd.: lauryl polyglyceryl-3 polydimethylsiloxyethyl dimethicone (Note 5) Manufactured by Shin-Etsu Chemical Co., Ltd.: methicone treatment (Note 6) Manufactured by Shin-Etsu Chemical Co., Ltd.: colored inorganic pigment treated by triethoxysilylethylpolydimethylsiloxyethylhexyldimethico ne, W: white, R: red, Y: yellow, B: black | |

### (Manufacturing method)

A: Components 1 to 9 were heated and dissolved.
B: Mix components 12 to 18 uniformly, and perform roll-treatment.
C: Add the material obtained in step B above and components 10 to 11 to the heated material obtained in step A above and mix uniformly.
D: After defoaming the product obtained in step C above, it is filled into a container with heating and cooled down to room temperature to obtain oil-based solid foundation.

The inventive oil-based solid foundation obtained as described above spread easily, had good fitting to the skin, gave a moist finish, provided firm cosmetic film with a suppressed shine, had good cosmetic persistence, and was extremely excellent.

### Example 16: Oil-based foundation

| Component | (%) |
|---|---|
| 1. KSG-42A (Note 1) | 10.0 |
| 2. Composite particle obtained in Example 3 | 6.0 |
| 3. KF-6104 (Note 2) | 4.0 |
| 4. Disteardimonium hectorite | 1.5 |
| 5. Silylated silica | 1.0 |
| 6. TSPL-30-ID (Note 3) | 2.0 |
| 7. KF-96A-6cs (Note 4) | 5.0 |
| 8. Ethanol | 8.0 |
| 9. Isododecane | Residual amount |
| 10. Isotridecyl isononanoate | 10.0 |
| 11. KP-578 (Note 5) | 0.5 |
| 12. KF-9901 treated titanium oxide fine particle (Note 6) | 8.0 |
| 13. KF-9901 treated zinc oxide fine particle (Note 6) | 5.0 |
| 14. AES-3083 treated pigment grade titanium oxide (Note 7) | |
| 15. AES-3083 treated iron oxide (red) (Note 7) | 0.4 |
| 16. AES-3083 treated iron oxide (yellow) (Note 7) | 7.5 1.2 100.0 |
| 17. AES-3083 treated iron oxide (black) (Note 7) | 0.1 |
| Total | |

| | |
|---|---|
| (Note 1) Manufactured by Shin-Etsu Chemical Co., Ltd.: mixture of (isododecane 75-85% + (vinyl dimethicone/lauryl dimethicone) crosspolymer 15-25%) (Note 2) Manufactured by Shin-Etsu Chemical Co., Ltd.: polyglyceryl-3 polydimethylsiloxyethyl dimethicone (Note 3) Manufactured by Shin-Etsu Chemical Co., Ltd.: dissolved product of 30% pullulan tri(trimethylsiloxy)silylpropylcarbamate in isododecane (Note 4) Manufactured by Shin-Etsu Chemical Co., Ltd.: dimethicone with a kinematic viscosity of 6 mm²/s at 25°C (Note 5) Manufactured by Shin-Etsu Chemical Co., Ltd.: (acrylates/ethylhexyl acrylate/dimethicone methacrylate) copolymer (Note 6) Manufactured by Shin-Etsu Chemical Co., Ltd.: hydrogen dimethicone treatment (Note 7) Manufactured by Shin-Etsu Chemical Co., Ltd.: triethoxycaprylylsilane treatment | |

### (Manufacturing method)

A: Mix components 1 to 9 uniformly.
B: Mix components 10 to 17 uniformly, and perform roll-treatment.
C: Add the material obtained in step B above to the material obtained in step A above and mix uniformly.
D: After defoaming the product obtained in step C above, it is filled into a container to obtain oil-based foundation.

The inventive oil-based foundation obtained as described above spread easily, had good fitting to the skin, gave a moist finish, provided firm cosmetic film with a suppressed shine, had good cosmetic persistence, and was extremely excellent.

### Example 17: Oil-based mascara

| Component | (%) |
|---|---|
| 1. Paraffin wax | 20.0 |
| 2. Microcrystalline wax | 8.0 |
| 3. Polyethylene wax | 3.0 |
| 4. Inulin stearate | 1.0 |
| 5. Disteardimonium hectorite | 2.0 |
| 6. KF-6028 (Note 1) | 1.0 |
| 7. NBN-30-ID (Note 2) | 3.0 |
| 8. Hydrogenated polyisobutene | Residual amount |
| 9. Composite particle obtained in Example 3 | 2.0 |
| 10. Neopentyl glycol dicaprate | 5.0 |
| 11. KF-6115 (Note 3) | 1.0 |
| 12. KTP-09W (Note 4) | 0.5 |
| 13. KTP-09B (Note 4) | 6.0 |
| Total | 100.0 |

| | |
|---|---|
| (Note 1) Manufactured by Shin-Etsu Chemical Co., Ltd.: PEG-9 polydimethylsiloxyethyl dimethicone (Note 2) Manufactured by Shin-Etsu Chemical Co., Ltd.: dissolved product of 30% norbornene/tris(trimethylsiloxy)silylnorbornene copolymer in isododecane (Note 3) Manufactured by Shin-Etsu Chemical Co., Ltd.: lauryl polyglyceryl-3 polydimethylsiloxyethyl dimethicone (Note 4) Manufactured by Shin-Etsu Chemical Co., Ltd.: colored inorganic pigment treated by triethoxysilylethylpolydimethylsiloxyethylhexyldimethico ne, W: white, B: black | |

### (Manufacturing method)

A: Components 1 to 8 were heated and dissolved.
B: Mix components 10 to 13 uniformly, and perform roll-treatment.
C: Add the material obtained in step B above and component 9 to the heated material obtained in step A above and mix uniformly.
D: After defoaming the product obtained in step C above, it is cooled down to room temperature and filled into a container to obtain oil-based mascara.

It was found that the inventive oil-based mascara obtained as described above had excellent smoothness in applying, was not sticky, spread easily, was excellent in adhesiveness, had good fitting, gave a finish with a suppressed shine, had good cosmetic persistence without bleeding, and was extremely excellent.

### Example 18: Loose powder

| Component | (%) |
|---|---|
| 1. Composite particle obtained in Example 2 | 10.0 |
| 2. KF-9901 treated synthesis fluorophlogopite (Note 1) | 25.0 |
| 3. KF-9901 treated talc (Note 1) | Residual amount |
| 4. Lauroyl lysine | 5.0 |
| 5. Boron nitride | 3.0 |
| 6. Pearl agent of mica coated with titanium oxide | 3.0 |
| 7. AES-3083 treated titanium oxide (Note 2) | 3.0 |
| 8. AES-3083 treated iron oxide (red) (Note 2) | 0.2 |
| 9. AES-3083 treated iron oxide (yellow) (Note 2) | 0.5 |
| 10. AES-3083 treated iron oxide (black) (Note 2) | 0.1 |
| 11. Isononyl isononanoate | 2.0 |
| 12. Ethylhexylglycerin | 0.5 |
| Total | 100.0 |

| | |
|---|---|
| (Note 1) Manufactured by Shin-Etsu Chemical Co., Ltd.: hydrogen dimethicone treatment (Note 2) Manufactured by Shin-Etsu Chemical Co., Ltd.: triethoxycaprylylsilane treatment | |

### (Manufacturing method)

A: Mix components 1 to 10 uniformly.
B: Mix components 11 to 12 uniformly.
C: Add the material obtained in step B above to the material obtained in step A above and mix uniformly.
D: Material obtained in step C above was passed through a sieve and filled into a container to obtain loose powder.

It was found that the inventive loose powder obtained as described above had excellent smoothness in applying, gave a finish with a suppressed shine, had good cosmetic persistence, and was extremely excellent.

### Example 19: Sunscreen emulsion

| Component | (%) |
|---|---|
| 1. KSG-270 (Note 1) | 3.0 |
| 2. KSG-18A (Note 2) | 3.0 |
| 3. KF-6048 (Note 3) | 2.0 |
| 4. KF-56A (Note 4) | 5.0 |
| 5. KF-4422 (Note 5) | Residual amount |
| 6. Ethylhexyl methoxycinnamate | 5.0 |
| 7. Ethylhexyl salicylate | 2.0 |
| 8. Octocrylene | 1.0 |
| 9. Diethylamino hydroxybenzoyl hexyl benzoate | 2.0 |
| 10. Composite particle obtained in Example 1 | 5.0 |
| 11. SPD-T5 (Note 6) | 10.0 |
| 12. SPD-Z5 (Note 7) | 10.0 |
| 13. BG | 3.0 |
| 14. Ethanol | 5.0 |
| 15. Sodium citrate | 0.2 |
| 16. Sodium chloride | 0.5 |
| 17. Water | 20.0 |
| Total | 100.0 |

| | |
|---|---|
| (Note 1) Manufactured by Shin-Etsu Chemical Co., Ltd.: mixture of (diphenylsiloxyphenyl trimethicone 75-85% + (dimethicone/(PEG-10/15)) crosspolymer 15-25%) (Note 2) Manufactured by Shin-Etsu Chemical Co., Ltd.: mixture of (diphenylsiloxyphenyl trimethicone 80-90% + (dimethicone/phenyl vinyl dimethicone) crosspolymer 10-20%) (Note 3) Manufactured by Shin-Etsu Chemical Co., Ltd.: cetyl PEG/PPG-10/1 Dimethicone (Note 4) Manufactured by Shin-Etsu Chemical Co., Ltd.: diphenylsiloxyphenyl trimethicone (Note 5) Manufactured by Shin-Etsu Chemical Co., Ltd.: ethyl trimethicone (Note 6) Manufactured by Shin-Etsu Chemical Co., Ltd.: cyclopentasiloxane dispersion of 40% titanium oxide fine particles (Note 7) Manufactured by Shin-Etsu Chemical Co., Ltd.: cyclopentasiloxane dispersion of 60% zinc oxide fine particles | |

### (Manufacturing method)

A: Mix components 1 to 10 uniformly.
B: Mix components 13 to 17 uniformly.
C: Add the material obtained in step B above to the material obtained in step A above and mix uniformly.
D: Add components 11 to 12 to the material obtained in step C above and mix uniformly.
E: After defoaming the product obtained in step D above, it is filled into a container to obtain sunscreen emulsion.

It was found that the inventive sunscreen emulsion obtained as described above had excellent smoothness in applying, gave a finish with a suppressed shine, had good cosmetic persistence, and was extremely excellent.

### Example 20: Sunscreen emulsion

| Component | (%) |
|---|---|
| 1. KSG-210 (Note 1) | 3.5 |
| 2. KSG-18A (Note 2) | 3.0 |
| 3. KF-6038 (Note 3) | 0.5 |
| 4. KF-56A (Note 4) | 5.0 |
| 5. Ethylhexyl methoxycinnamate | 7.5 |
| 6. KF-995 (Note 5) | 6.5 |
| 7. Composite particle obtained in Example 3 | 5.0 |
| 8. BG | 5.5 |
| 9. Sodium citrate | 0.2 |
| 10. Sodium chloride | 0.5 |
| 11. Water | 62.8 |
| Total | 100.0 |

| | |
|---|---|
| (Note 1) Manufactured by Shin-Etsu Chemical Co., Ltd.: mixture of (dimethicone 70-80% + (dimethicone/(PEG-10/15)) crosspolymer 20-30%) (Note 2) Manufactured by Shin-Etsu Chemical Co., Ltd.: mixture of (diphenylsiloxyphenyl trimethicone 80-90% + (dimethicone/phenyl vinyl dimethicone) crosspolymer 10-20%) (Note 3) Manufactured by Shin-Etsu Chemical Co., Ltd.: lauryl PEG-9 polydimethylsiloxyethyl dimethicone (Note 4) Manufactured by Shin-Etsu Chemical Co., Ltd.: diphenylsiloxyphenyl trimethicone (Note 5) Manufactured by Shin-Etsu Chemical Co., Ltd.: cyclopentasiloxane | |

### (Manufacturing method)

A: Mix components 1 to 7 uniformly.
B: Mix components 8 to 11 uniformly.
C: Add the material obtained in step B above to the material obtained in step A above and mix uniformly.
D: After defoaming the product obtained in step C above, it is filled into a container to obtain sunscreen emulsion.

It was found that the inventive sunscreen emulsion obtained as described above had excellent smoothness in applying, gave a finish with a suppressed shine, had good cosmetic persistence, and was extremely excellent.

### Example 21: Nonaqueous deodorant cream

| Component | (%) |
|---|---|
| 1. Trichlorohydrex glycine (Al/zirconium) | 19.0 |
| 2. KF-4422 (Note 1) | 30.0 |
| 3. KSG-15 (Note 2) | 21.5 |
| 4. Composite particle obtained in Example 3 | 10.0 |
| 5. Neopentyl glycol dioctoate | 9.68 |
| 6. Fragrance | 0.1 |
| 7. BHT | 0.02 |
| 8. Citric acid | 0.1 |
| 9. Benzyl alcohol | 0.1 |
| 10. Dimethylsilylated silica | 0.5 |
| 11. Polyethylene | 3.0 |
| 12. Ceresin | 6.0 |
| Total | 100.0 |

| | |
|---|---|
| (Note 1) Manufactured by Shin-Etsu Chemical Co., Ltd.: ethyl trimethicone (Note 2) Manufactured by Shin-Etsu Chemical Co., Ltd.: mixture of (cyclopentasiloxane 90-96% + (dimethicone/vinyl dimethicone) crosspolymer 4-10%) | |

### (Manufacturing method)

A: Components 2, 3, 5, 7, 9, 11, and 12 were heated, and mixed uniformly.
B: Add component 10 to the material obtained in step A above and the mixture was dispersed and mixed uniformly using a mixer.
C: Add components 1, 4, and 8 to the material obtained in step B above and mix uniformly.
D: Add component 6 to the material obtained in step C above, mix uniformly, and it is filled into a container.

The deodorant cream obtained as described above had excellent smoothness in applying and spreading, spread easily, was not excessively dry and sticky, was nonaqueous deodorant cream having good persistence of deodorizing effect.

As described above, the present invention can provide: a composite particle comprising an organic resin particle having a rubber particle adhered on a surface of the organic resin particle, the composite particle that can give effect of a soft feeling to the touch, a smooth feeling in use, spreadability, a natural finish thanks to light scattering properties; a manufacturing method for the composite particle; and cosmetic comprising the composite particle.

The present description includes the following embodiments.
[1]: A composite particle comprising: an organic resin particle having a spherical shape; and a rubber particle adhered on a surface of the organic resin particle,
   wherein the rubber particle is fixed to the organic resin particle using silica as a binder.
[2]: The composite particle according to the above [1],
   wherein content of the rubber particle is within a range of 0.1 to 100 parts by mass relative to 100 parts by mass of the organic resin particle.
[3]: The composite particle according to the above [1] or [2],
   wherein content of the silica is within a range of 10 to 1,000 parts by mass relative to 100 parts by mass of the rubber particle.
[4]: The composite particle according to the above [1], [2], or [3],
   wherein the rubber particle is a silicone rubber particle.
[5]: The composite particle according to the above [1], [2], [3], or [4],
   wherein the organic resin particle is a cellulose particle.
[6]: A method for manufacturing the composite particle according to the above [1], [2], [3] ,[4], or [5],
   the method comprising adding tetraalkoxysilane to a liquid mixture of the organic resin particle, the rubber particle, a cationic substance, an alkaline substance, and water, and subjecting the tetraalkoxysilane to a hydrolysis/condensation reaction,
   wherein the cationic substance is a cationic surfactant and/or a cationic water-soluble polymer.
[7]: The method for manufacturing the composite particle according to the above [6],
   wherein the cationic substance is a cationic surfactant and a cationic water-soluble polymer.
[8]: The method for manufacturing the composite particle according to the above [6] or [7],
   wherein a blending amount of the cationic substance is within a range of 0.0001 to 2.0 parts by mass relative to 100 parts by mass of the water in the liquid mixture.
[9]: The method for manufacturing the composite particle according to the above [6], [7], or [8],
   wherein a blending amount of the cationic surfactant is within a range of 0.001 to 1.9 parts by mass relative to 100 parts by mass of the water in the liquid mixture, and a blending amount of the cationic water-soluble polymer is within a range of 0.0001 to 1.0 parts by mass relative to 100 parts by mass of the water in the liquid mixture.
[10]: A cosmetic comprising the composite particle according to the above [1], [2], [3], [4], or [5].

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A composite particle comprising: an organic resin particle having a spherical shape; and a rubber particle adhered on a surface of the organic resin particle,
wherein the rubber particle is fixed to the organic resin particle using silica as a binder.

2. The composite particle according to claim 1,
wherein content of the rubber particle is within a range of 0.1 to 100 parts by mass relative to 100 parts by mass of the organic resin particle.

3. The composite particle according to claim 1,
wherein content of the silica is within a range of 10 to 1,000 parts by mass relative to 100 parts by mass of the rubber particle.

4. The composite particle according to claim 1,
wherein the rubber particle is a silicone rubber particle.

5. The composite particle according to any one of claims 1 to 4,
wherein the organic resin particle is a cellulose particle.

6. A method for manufacturing the composite particle according to claim 1,
the method comprising adding tetraalkoxysilane to a liquid mixture of the organic resin particle, the rubber particle, a cationic substance, an alkaline substance, and water, and subjecting the tetraalkoxysilane to a hydrolysis/condensation reaction,
wherein the cationic substance is a cationic surfactant and/or a cationic water-soluble polymer.

7. The method for manufacturing the composite particle according to claim 6,
wherein the cationic substance is a cationic surfactant and a cationic water-soluble polymer.

8. The method for manufacturing the composite particle according to claim 6,
wherein a blending amount of the cationic substance is within a range of 0.0001 to 2.0 parts by mass relative to 100 parts by mass of the water in the liquid mixture.

9. The method for manufacturing the composite particle according to any one of claims 6 to **8,**
wherein a blending amount of the cationic surfactant is within a range of 0.001 to **1.9** parts by mass relative to 100 parts by mass of the water in the liquid mixture, and a blending amount of the cationic water-soluble polymer is within a range of 0.0001 to 1.0 parts by mass relative to 100 parts by mass of the water in the liquid mixture.

10. A cosmetic comprising the composite particle according to any one of claims 1 to 4.
